(19) Europäisches Patentamt
European Patent Office
Office européen des brevets

(11) EP 1 606 633 B1

(12) **EUROPEAN PATENT SPECIFICATION**

(45) Date of publication and mention of the grant of the patent:
**04.06.2008 Bulletin 2008/23**

(21) Application number: **04717063.4**

(22) Date of filing: **04.03.2004**

(51) Int Cl.:
***G01N 33/68*** (2006.01) ***C12Q 1/68*** (2006.01)
***A61K 38/00*** (2006.01) ***A61K 39/00*** (2006.01)

(86) International application number:
**PCT/EP2004/002183**

(87) International publication number:
**WO 2004/082569 (30.09.2004 Gazette 2004/40)**

(54) **DIAGNOSTICS AND THERAPEUTICS FOR DISEASES ASSOCIATED WITH DOPAMINE RECEPTOR D3 (DRD3)**

DIAGNOSTIK- UND THERAPIE- VERFAHREN VON MIT DOPAMINREZEPTOR D3 (DRD3) VERBUNDENEN KRANKHEITEN

DIAGNOSTICS ET TRAITEMENTS CONTRE DES MALADIES ASSOCIEES AU RECEPTEUR D3 DE LA DOPAMINE (DRD3)

(84) Designated Contracting States:
**AT BE BG CH CY CZ DE DK EE ES FI FR GB GR HU IE IT LI LU MC NL PL PT RO SE SI SK TR**

(30) Priority: **17.03.2003 EP 03005465**

(43) Date of publication of application:
**21.12.2005 Bulletin 2005/51**

(73) Proprietor: **Bayer HealthCare AG**
**51368 Leverkusen (DE)**

(72) Inventors:
- **GOLZ, Stefan**
  **45326 Essen (DE)**
- **BRÜGGEMEIER, Ulf**
  **42799 Leichlingen (DE)**
- **SUMMER, Holger**
  **42113 Wuppertal (DE)**

(56) References cited:
**WO-A-00/42036** **WO-A1-94/20079**
**DE-A- 19 922 443** **US-A- 5 407 823**

- **ASICO L D ET AL: "DISRUPTION OF THE DOPAMINE D3 RECEPTOR GENE PRODUCES RENIN-INDEPENDENT HYPERTENSION" JOURNAL OF CLINICAL INVESTIGATION, NEW YORK, NY, US, vol. 102, no. 3, 1 August 1996 (1996-08-01), pages 493-498, XP000986718 ISSN: 0021-9738 cited in the application**
- **LIEBMAN M.N. DRUG DISCOVERY TODAY vol. 7, no. 20(SUPPL.), 2002, pages S197 - S203**
- **FEHNIGER T.E. ET AL DRUG DISCOVERY TODAY: TECHNOLOGIES vol. 2, no. 4, 2005, pages 345 - 351**
- **MC ALLISTER ET AL J.RECEPT. SIGNAL TRANSDUCTION RES. vol. 15, 1995, pages 267 - 281**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 606 633 B1

SEQUENCE LISTING

<110> Bayer AG, BHC
<120> Diagnostics and Therapeutics for Diseases Associated with DOPAMINE RECEPTOR D3 (DRD3)
<130> Le A 36 640
<160> 5
<170> Patent In version 3.1
<210> 1
<211> 1203
<212> DNA
<213> Homo sapiens
<400> 1

```
atggcatctc tgagtcagct gagtagccac ctgaactaca cctgtggggc agagaactcc     60
acaggtgcca gccaggcccg cccacatgcc tactatgccc tctcctactg cgcgctcatc    120
ctggccatcg tcttcggcaa tggcctggtg tgcatggctg tgctgaagga gcgggccctg    180
cagactacca ccaactactt agtagtgagc ctggctgtgg cagacttgct ggtggccacc    240
ttggtgatgc cctgggtggt atacctggag gtgacaggtg gagtctggaa tttcagccgc    300
atttgctgtg atgtttttgt caccctggat gtcatgatgt gtacagccag catccttaat    360
ctctgtgcca tcagcataga caggtacact gcagtggtca tgcccgttca ctaccagcat    420
ggcacgggac agagctcctg tcggcgcgtg gccctcatga tcacggccgt ctgggtactg    480
gcctttgctg tgtcctgccc tcttctgttt ggctttaata ccacagggga ccccactgtc    540
tgctccatct ccaaccctga ttttgtcatc tactcttcag tggtgtcctt ctacctgccc    600
tttggagtga ctgtccttgt ctatgccaga atctatgtgg tgctgaaaca aaggagacgg    660
aaaaggatcc tcactcgaca gaacagtcag tgcaacagtg tcaggcctgg cttcccccaa    720
caaaccctct ctcctgaccc ggcacatctg gagctgaagc gttactacag catctgccag    780
gacactgcct tgggtggacc aggcttccaa gaaagaggag gagagttgaa aagagaggag    840
aagactcgga attccctgag tcccaccata gcgcccaagc tcagcttaga agttcgaaaa    900
ctcagcaatg gcagattatc gacatctttg aagctggggc ccctgcaacc tcggggagtg    960
ccacttcggg agaagaaggc aacccaaatg gtggccattg tgcttggggc cttcattgtc   1020
tgctggctgc ccttcttctt gacccatgtt ctcaataccc actgccagac atgccacgtg   1080
tccccagagc tttacagtgc cacgacatgg ctgggctacg tgaatagcgc cctcaaccct   1140
gtgatctata ccaccttcaa tatcgagttc cggaaagcct tcctcaagat cctgtcttgc   1200
tga                                                                 1203
```

<210> 2
<211> 400
<212> PRT
<213> Homo sapiens
<400> 2

```
Met Ala Ser Leu Ser Gln Leu Ser Ser His Leu Asn Tyr Thr Cys Gly
1               5                   10                  15
Ala Glu Asn Ser Thr Gly Ala Ser Gln Ala Arg Pro His Ala Tyr Tyr
            20                  25                  30
Ala Leu Ser Tyr Cys Ala Leu Ile Leu Ala Ile Val Phe Gly Asn Gly
        35                  40                  45
```

Leu Val Cys Met Ala Val Leu Lys Glu Arg Ala Leu Gln Thr Thr Thr
50 55 60
Asn Tyr Leu Val Val Ser Leu Ala Val Ala Asp Leu Leu Val Ala Thr
65 70 75 80
Leu Val Met Pro Trp Val Val Tyr Leu Glu Val Thr Gly Gly Val Trp
85 90 95
Asn Phe Ser Arg Ile Cys Cys Asp Val Phe Val Thr Leu Asp Val Met
100 105 110
Met Cys Thr Ala Ser Ile Leu Asn Leu Cys Ala Ile Ser Ile Asp Arg
115 120 125
Tyr Thr Ala Val Val Met Pro Val His Tyr Gln His Gly Thr Gly Gln
130 135 140
Ser Ser Cys Arg Arg Val Ala Leu Met Ile Thr Ala Val Trp Val Leu
145 150 155 160
Ala Phe Ala Val Ser Cys Pro Leu Leu Phe Gly Phe Asn Thr Thr Gly
165 170 175
Asp Pro Thr Val Cys Ser Ile Ser Asn Pro Asp Phe Val Ile Tyr Ser
180 185 190
Ser Val Val Ser Phe Tyr Leu Pro Phe Gly Val Thr Val Leu Val Tyr
195 200 205
Ala Arg Ile Tyr Val Val Leu Lys Gln Arg Arg Arg Lys Arg Ile Leu
210 215 220
Thr Arg Gln Asn Ser Gln Cys Asn Ser Val Arg Pro Gly Phe Pro Gln
225 230 235 240
Gln Thr Leu Ser Pro Asp Pro Ala His Leu Glu Leu Lys Arg Tyr Tyr
245 250 255
Ser Ile Cys Gln Asp Thr Ala Leu Gly Gly Pro Gly Phe Gln Glu Arg
260 265 270
Gly Gly Glu Leu Lys Arg Glu Glu Lys Thr Arg Asn Ser Leu Ser Pro
275 280 285
Thr Ile Ala Pro Lys Leu Ser Leu Glu Val Arg Lys Leu Ser Asn Gly
290 295 300
Arg Leu Ser Thr Ser Leu Lys Leu Gly Pro Leu Gln Pro Arg Gly Val
305 310 315 320
Pro Leu Arg Glu Lys Lys Ala Thr Gln Met Val Ala Ile Val Leu Gly
325 330 335
Ala Phe Ile Val Cys Trp Leu Pro Phe Phe Leu Thr His Val Leu Asn
340 345 350
Thr His Cys Gln Thr Cys His Val Ser Pro Glu Leu Tyr Ser Ala Thr
355 360 365
Thr Trp Leu Gly Tyr Val Asn Ser Ala Leu Asn Pro Val Ile Tyr Thr
370 375 380
Thr Phe Asn Ile Glu Phe Arg Lys Ala Phe Leu Lys Ile Leu Ser Cys
385 390 395 400

<210> 3

<211> 20
<212> DNA
<213> artificial sequence
<220>
<223> forward primer
<400> 3
aaaccctctc tcctgacccg 20
<210> 4
<211> 19
<212> DNA
<213> artificial sequence
<220>
<223> reverse primer
<400> 4
acccaaggca gtgtcctgg 19
<210> 5
<211> 30
<212> DNA
<213> artificial sequence
<220>
<223> probe
<400> 5
cacatctgga gctgaagcgt tactacagca 30

## Description

### Technical field of the invention

The present invention is in the field of molecular biology, more particularly, the present invention relates to nucleic acid sequences and amino acid sequences of a human DRD3 and its regulation for the treatment of cardiovascular disorders in mammals comprised on the group of heart failure, ischemic diseases of the heart, myocardial infarction and peripheral vascular diseases.

### Background of the invention

#### G-Protein Coupled Receptors

DRD3 is a seven transmembrane G protein coupled receptor (GPCR) [WO920793, WO200177172, Sokoloff et al. (1990), Le Coniat et al. (1991), Crocq et al. (1992), Shaikh et al. (1993), Nothen et al. (1993), Nanko et al. (1993), Spurlock et al. (1998), Ilani et al. (2001), Holzman (2000), Rybakowski et al. (2001), Lohmueller et al. (2003), Accili et al. (1996), Asico et al. (1998), Pilla et al. (1999), Guillin et al. (2001)]. Many medically significant biological processes are mediated by signal transduction pathways that involve G-proteins [Lefkowitz, (1991)]. The family of G-protein coupled receptors (GPCRs) includes receptors for hormones, neurotransmitters, growth factors, and viruses. Specific examples of GPCRs include receptors for such diverse agents as dopamine, calcitonine, adrenergic hormones, endotheline, cAMP, adenosine, acetylcholine, serotonine, histamine, thrombin, kinine, follicle stimulating hormone, opsins, endothelial differentiation gene-1, rhodopsins, odorants, cytomegalovirus, G-proteins themselves, effector proteins such as phospholipase C, adenyl cyclase, and phosphodiesterase, and actuator proteins: such as protein kinase A and protein kinase C.

GPCRs possess seven conserved membrane-spanning domains connecting at least eight divergent hydrophilic loops. GPCRs, also known as seven transmembrane, 7TM, receptors, have been characterized as including these seven conserved hydrophobic stretches of about 20 to 30 amino acids, connecting at least eight divergent hydrophilic loops. Most GPCRs have single conserved cysteine residues in each of the first two extracellular loops, which form disulfide bonds that are believed to stabilize functional protein structure. The seven transmembrane regions are designated as TM1, TM2, TM3, TM4, TM5, TM6, and TM7. TM3 is being implicated with signal transduction. Phosphorylation and lipidation (palmitylation or farnesylation) of cysteine residues can influence signal transduction of some GPCRs. Most GPCRs contain potential phosphorylation sites within the third cytoplasmic loop and/or the carboxy terminus. For several GPCRs, such as the beta-adrenergic receptor, phosphorylation by protein kinase A and/or specific receptor kinases mediates receptor desensitization.

For some receptors, the ligand binding sites of GPCRs are believed to comprise hydrophilic sockets formed by several GPCR transmembrane domains. The hydrophilic sockets are surrounded by hydrophobic residues of the GPCRs. The hydrophilic side of each GPCR transmembrane helix is postulated to face inward and form a polar ligand binding site. TM3 is being implicated with several GPCRs as having a ligand binding site, such as the TM3 aspartate residue. TM5 serines, a TM6 asparagine, and TM6 or TM7 phenylalanines or tyrosines also are implicated in ligand binding.

GPCRs are coupled inside the cell by heterotrimeric G-proteins to various intracellular enzymes, ion channels, and transporters. Different G-protein alpha-subunits preferentially stimulate particular effectors to modulate various biological functions in a cell. Phosphorylation of cytoplasmic residues of GPCRs is an important mechanism for the regulation of some GPCRs. For example, in one form of signal transduction, the effect of hormone binding is the activation of the enzyme, adenylate cyclase, inside the cell. Enzyme activation by hormones is dependent on the presence of the nucleotide GTP. GTP also influences hormone binding. A G-protein connects the hormone receptor to adenylate cyclase. G-protein exchanges GTP for bound GDP when activated by a hormone receptor. The GTP-carrying form then binds to activated adenylate cyclase. Hydrolysis of GTP to GDP, catalyzed by the G-protein itself, returns the G-protein to its basal, inactive form. Thus, the G-protein serves a dual role, as an intermediate that relays the signal from receptor to effector, and as a clock that controls the duration of the signal.

Over the past 15 years, nearly 350 therapeutic agents targeting 7TM receptors have been successfully introduced into the market. This indicates that these receptors have an established, proven history as therapeutic targets. Clearly, there is a need for identification and characterization of further receptors which can play a role in preventing, ameliorating, or correcting dysfunctions or diseases including, but not limited to, infections such as bacterial, fungal, protozoan, and viral infections, particularly those caused by HIV viruses, cancers, allergies including asthma, cardiovascular diseases including acute heart failure, hypotension, hypertension, angina pectoris, myocardial infarction, hematological diseases, genito-urinary diseases including urinary incontinence and benign prostate hyperplasia, osteoporosis, and peripheral and central nervous system disorders including pain, Alzheimer's disease and Parkinson's disease.

**TaqMan-Technology / expression profiling**

TaqMan is a recently developed technique, in which the release of a fluorescent reporter dye from a hybridisation probe in real-time during a polymerase chain reaction (PCR) is proportional to the accumulation of the PCR product. Quantification is based on the early, linear part of the reaction, and by determining the threshold cycle (CT), at which fluorescence above background is first detected.

Gene expression technologies may be useful in several areas of drug discovery and development, such as target identification, lead optimization, and identification of mechanisms of action. The TaqMan technology can be used to compare differences between expression profiles of normal tissue and diseased tissue. Expression profiling has been used in identifying genes, which are up- or downregulated in a variety of diseases. An interesting application of expression profiling is temporal monitoring of changes in gene expression during disease progression and drug treatment or in patients versus healthy individuals. The premise in this approach is that changes in pattern of gene expression in response to physiological or environmental stimuli (e.g., drugs) may serve as indirect clues about disease-causing genes or drug targets. Moreover, the effects of drugs with established efficacy on global gene expression patterns may provide a guidepost, or a genetic signature, against which a new drug candidate can be compared.

WO 03094856 discloses targets for the therapy of cardiovascular diseases, such as heart failure and myocardial infarction.

**DRD3**

The nucleotide sequence of DRD3 is accessible in public databases by the accession number NM_000796 and is given in SEQ ID NO:1. The amino acid sequence of DRD3 is depicted in SEQ ID NO:2.

G protein-coupled receptors play a role in cell communication. They are characterized by an extracellular N terminus, 7 transmembrane regions, and an intracellular C terminus.

Sokoloff et al. (1990) characterized a dopamine receptor that differs in its pharmacology and signaling system from the D1 and D2 receptors and represents both an autoreceptor and a postsynaptic receptor. Designated the dopamine receptor D3, it is localized to limbic areas of the brain, which are associated with cognitive, emotional, and endocrine functions. By screening cDNA and genomic libraries using a combination of reverse transcription and PCR, Sokoloff et al. (1990) cloned the DRD3 gene.

Sokoloff et al. (1990) found that the DRD3 gene, like the DRD2 gene but unlike most other members of this superfamily, contains introns, 5 in number. The position of 2 of the introns corresponds to that of introns in DRD2.

Le Coniat et al. (1991) assigned the DRD3 gene to chromosome 3 by hybridization of a genomic probe to flow-sorted chromosomes and localized it to band 3q13.3 by in situ hybridization.

Sokoloff et al. (1990) noted that the D3 receptor appeared to mediate some of the effects of antipsychotic drugs and drugs used in the treatment of Parkinson disease, which were previously thought to interact only with D2 receptors.

Crocq et al. (1992) presented data from 2 independent studies carried out in the UK and France, determining frequencies of a Ball Poly- morphism in the DRD3 gene in patients with schizophrenia. In both studies, more patients than controls were homozygous (p =0.005, p = 0.008). When pooled data were analyzed, this difference was highly significant (p = 0.0001) with a relative risk of schizophrenia in homozygotes of 2.61 (95% CI = 1.60-4.26). Shaikh et al. (1993) failed to find similar results when they studied bipolar affective disorder.

They believed that this indicated a genetic distinction between the 2 disorders which have been thought by some to be different expressions of the same underlying disturbance. Nothen et al. (1993) were unable to confirm the finding by Crocq et al. (1992) of an association between schizophrenia and homozygosity at the DRD3 receptor locus. They pointed out that there was an overrepresentation of heterozygotes in the control groups studied by Crocq et al. (1992). In a study of 91 Japanese patients and 90 controls, Nanko et al. (1993) likewise could not confirm the findings of Crocq et al. (1992). However, Spurlock et al. (1998) did replicate the findings of Crocq et al. (1992) as part of the European Multicentre Association Study of Schizophrenia. An excess of homozygotes for both alleles of the DRD3 polymorphism was observed in schizophrenic patients (chi(2), 8.54, P = 0.003; odds ratio, 1.64, 95% CI, 1.18-2.29).

Although the precise pathophysiology of schizophrenia is unknown, the dopaminergic hypothesis assumes that the illness results from excessive activity at dopamine synapses in the brain. Because the diagnosis of schizophrenia relies on descriptive behavioral and symptomatic information, a peripheral measurable marker might enable a simpler, more rapid, and more accurate diagnosis and monitoring. Peripheral blood lymphocytes have been found to express several dopamine receptors: D3, D4 (DRD4), and D5 (DRD5). It has been suggested, furthermore, that these dopamine receptors found on lymphocytes reflect the receptors found in the brain. Ilani et al. (2001) demonstrated a correlation between the D3 dopamine receptor on lymphocytes and schizophrenia and showed a significant elevation of at least 2-fold in the mRNA level of the D3, but not of the D4, dopamine receptor in schizophrenic patients. The increase was not affected by different antipsychotic drug treatments. Moreover, nonmedicated patients exhibited the same pattern, indicating that this change was not a result of medical treatment. Ilani et al. (2001) proposed that increased D3 receptor mRNA on blood lymphocytes can be used as a marker for identification and follow-up of schizophrenia.

Eye movement disturbances occur in most patients with schizophrenia and in many of their healthy first-degree relatives (Holzman, 2000).

Rybakowski et al. (2001) studied fixation and smooth pursuit eye abnormalities in 119 schizophrenic patients and 94 unrelated healthy control subjects in association with the ser9-to-gly polymorphism of the DRD3 gene. They found that both kinds of eye movement abnormalities were higher in individuals with a homozygous ser9 genotype. The ser9/ser9 genotype was more prevalent in patients with a higher intensity of both fixation (58.1 vs 23.9%, P less than 0.001) and smooth pursuit disturbances (52.3 vs 25.8%, P less than 0.02) and the ser9/gly9 genotype frequency was lower in patients with higher fixation disturbances (37.0 vs 60.9%, P less than 0.02).

Similarly, genotype frequency of ser9/ser9 was higher in subjects with any degree of eye movement disturbance than in control subjects. Rybakowski et al. (2001) suggested that the ser9/ser9 polymorphism may contribute to eye movement disturbances, which are used as a marker for schizophrenia.

Lohmueller et al. (2003) performed a metaanalysis of 301 published genetic association studies covering 25 different reported associations with common disorders. For 8 of these associations, pooled analysis of follow-up studies yielded statistically significant replication of the first report, with modest estimated genetic effects. One of these 8 was the DRD3/schizophrenia association (S/S of S9G polymorphism) as first reported by Crocq et al. (1992).

The role of the D3 receptor is difficult to study because of its low abundance (approximately 1% of D2 receptors) and the absence of selective ligands. Using a strategy of gene targeting in mouse embryonic stem cells, Accili et al. (1996) generated DRD3-deficient mice carrying a premature chain-termination mutation after residue arginine 148. Binding of the dopamine antagonist iodosulpride to D3 receptors was absent in mice homozygous for the mutation and greatly reduced in heterozygous mice. Behavioral analysis of mutant mice showed that this mutation is associated with hyperactivity. Homozygous mice lacking D3 receptors displayed increased locomotor activity and rearing behavior. Mice heterozygous for the mutation showed similar, although less pronounced, behavioral alterations.

Because dopamine receptors are important in the regulation of renal and cardiovascular function, Asico et al. (1998) studied the cardiovascular consequences of disruption of the D3 receptor, which is expressed in renal proximal tubules and juxtaglomerular cells, in the mouse. Systolic and diastolic blood pressures were higher (approximately 20 mmHg) in heterozygous and homozygous mice than in wildtype mice. An acute saline load increased urine flow rate and sodium excretion to a similar extent in wildtype and heterozygous mice but the increase was attenuated in homozygous mice. Renal renin activity was much greater in homozygous than in wildtype mice; values for heterozygous mice were intermediate. Blockade of angiotensin II subtype 1 receptors decreased systolic blood pressure for a longer duration in mutant than in wildtype mice. Thus, disruption of the D3 receptor increases renal renin production and produces renal sodium retention and renin-dependent-hypertension.

Pilla et al. (1999) identified a compound called BP897, which has a high affinity for the D3 receptor and a 70-times-lower affinity for the D2 receptor, as well as low affinities for the D1 and D4 receptors. BP897 is the first D3 receptor-selective agonist assessed in vitro with recombinant receptors and in vivo with mice bearing disrupted D3-receptor genes. BP897 is a partial agonist in vitro and acts in vivo as either an agonist or as a antagonist. Pilla et al. (1999) showed that BP897 inhibits cocaine-seeking behavior that depends upon the presentation of drug-associated cues, without having any intrinsic primary rewarding effects. The authors concluded that their data indicates that compounds like BP897 could be used for reducing the drug craving and vulnerability to relapse that are elicited by drug-associated environmental stimuli.

Brain-derived neurotrophic factor (BDNF) was initially regarded to be responsible for neuron proliferation, differentiation, and survival through its uptake at nerve terminals and retrograde transport to the cell body. A more diverse role for BDNF has emerged progressively from observations showing that it is also transported anterogradely, is released upon neuron depolarization, and triggers rapid intracellular signals and action potentials in central neurons. Guillin et al. (2001) reported that BDNF elicits long-term neuronal adaptations by controlling the responsiveness of its target neurons to the important neurotransmitter, dopamine. Using lesions and gene targeted mice lacking BDNF, Guillin et al. (2001) showed that BDNF from dopamine neurons is responsible for inducing normal expression of the dopamine D3 receptor in nucleus accumbens both during development and in adulthood. BDNF from corticostriatal neurons also induces behavioral sensitization by triggering overexpression of the D3 receptor in striatum of hemiparkinsonian rats. Guillin et al. (2001) concluded that BDNF may be an important determinant of pathophysiologic conditions such as drug addiction, schizophrenia, or Parkinson disease, in which D3 receptor expression is abnormal.

DRD3 shows the highest homology (55%) to the human Dopamine D2 receptor as shown in example 1. DRD3 is published in WO9207937 and WO200177172.

Mc Allister et. al. (1995) showed that the activation of DRD3 resulted in the inhibition of forskolinstimulated adenylate cyclase activity. Modulation of cellular cAMP levels leads to the modulation of ion channel gating resulting in an altered $Ca^{2+}$-ion flux, which is a welt known mechanism for cardiovascular function.

## Summary of the invention

The invention relates to novel disease associations of DRD3 polypeptides and polynucleotides. The invention also relates to novel methods of screening for therapeutic agents for the treatment of heart failure, ischemic diseases of the heart, myocardial infarction and peripheral vascular diseases The invention also relates to pharmaceutical compositions for the treatment of heart failure, ischemic diseases of the heart, myocardial infarction and peripheral vascular diseases in a mammal comprising a DRD3 polypeptide, a DRD3 polynucleotide, or, an antibody, a ribozyme or an antisense oligonucleotide which regulate DRD3 polypeptide activity.

## Brief Description of the Drawings

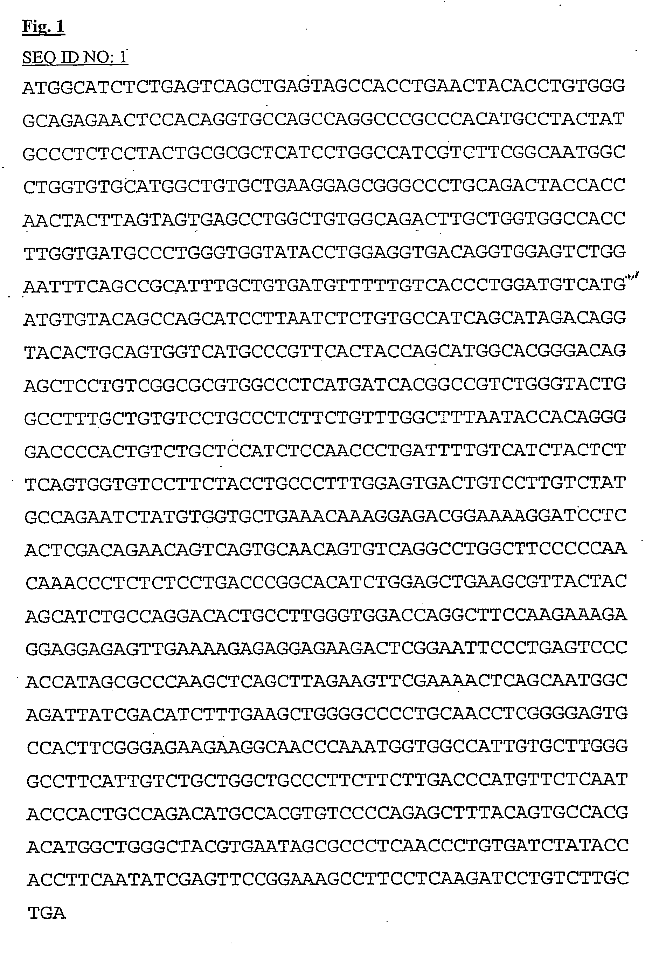

Fig. 1 shows the nucleotide sequence of a DRD3 receptor polynucleotide (SEQ ID NO:1).

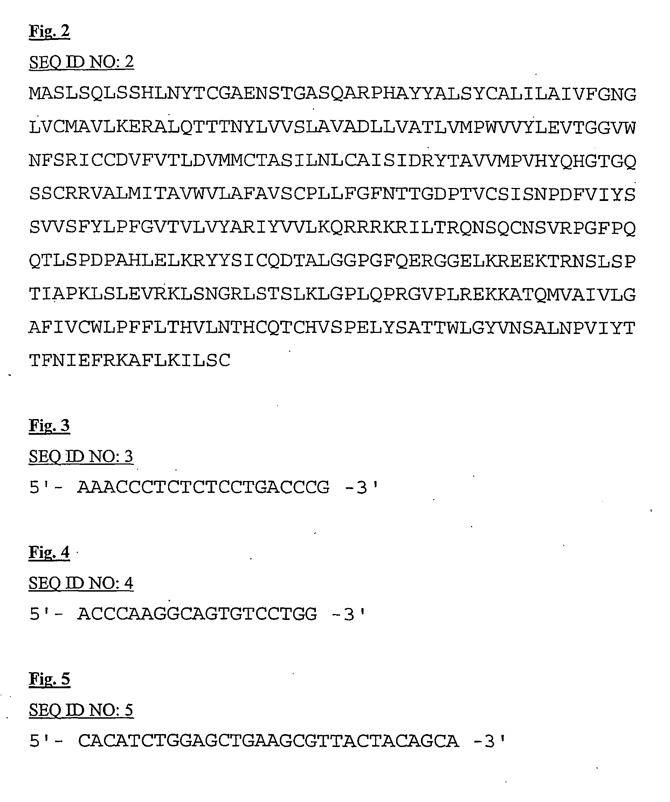

Fig. 2 shows the amino acid sequence of a DRD3 receptor polypeptide (SEQ ID NO:2).

Fig. 3 shows the nucleotide sequence of a primer useful for the invention (SEQ ID NO:3).

Fig. 4 shows the nucleotide sequence of a primer useful for the invention (SEQ ID NO:4).

Fig. 5 shows a nucleotide sequence useful as a probe to detect proteins of the invention (SEQ ID NO:5).

## Detailed description of the invention

### Definition of terms

An "oligonucleotide" is a stretch of nucleotide residues which has a sufficient number of bases to be used as an oligomer, amplimer or probe in a polymerase chain reaction (PCR). Oligonucleotides are prepared from genomic or cDNA sequence and are used to amplify, reveal, or confirm the presence of a similar DNA or RNA in a particular cell or tissue. Oligonucleotides or oligomers comprise portions of a DNA sequence having at least about 10 nucleotides and as many as about 35 nucleotides, preferably about 25 nucleotides.

"Probes" may be derived from naturally occurring or recombinant single- or double-stranded nucleic acids or may be chemically synthesized. They are useful in detecting the presence of identical or similar sequences. Such probes may be labeled with reporter molecules using nick translation, Klenow fill-in reaction, PCR or other methods well known in the art. Nucleic acid probes may be used in southern, northern or in situ hybridizations to determine whether DNA or RNA encoding a certain protein is present in a cell type, tissue, or organ.

A "fragment of a polynucleotide" is a nucleic acid that comprises all or any part of a given nucleotide molecule, the fragment having fewer nucleotides than about 6 kb, preferably fewer than about 1 kb.

"Reporter molecules" are radionuclides, enzymes, fluorescent, chemiluminescent, or chromogenic agents which associate with a particular nucleotide or amino acid sequence, thereby establishing the presence of a certain sequence, or allowing for the quantification of a certain sequence.

"Chimeric" molecules may be constructed by introducing all or part of the nucleotide sequence used in this invention into a vector containing additional nucleic acid sequence which might be expected to change any one or several of the following DRD3 characteristics: cellular location, distribution, ligand-binding affinities, interchain affinities, degradation/turnover rate, signaling, etc.

"Active", with respect to a DRD3 polypeptide, refers to those forms, fragments, or domains of a DRD3 polypeptide which retain the biological and/or antigenic activity of a DRD3 polypeptide.

"Naturally occurring DRD3 polypeptide" refers to a polypeptide produced by cells which have not been genetically engineered and specifically contemplates various polypeptides arising from post-translational modifications of the polypeptide including but not limited to acetylation, carboxylation, glycosylation, phosphorylation, lipidation and acylation.

"Derivative" refers to polypeptides which have been chemically modified by techniques such as ubiquitination, labeling (see above), pegylation (derivatization with polyethylene glycol), and chemical insertion or substitution of amino acids such as ornithine which do not normally occur in human proteins.

"Conservative amino acid substitutions" result from replacing one amino acid with another having similar structural and/or chemical properties, such as the replacement of a leucine with an isoleucine or valine, an aspartate with a glutamate, or a threonine with a serine.

"Insertions" or "deletions" are typically in the range of about 1 to 5 amino acids. The variation allowed may be experimentally determined by producing the peptide synthetically while systematically making insertions, deletions, or substitutions of nucleotides in the sequence using recombinant DNA techniques.

A "signal sequence" or "leader sequence" can be used, when desired, to direct the polypeptide through a membrane of a cell. Such a sequence may be naturally present on the polypeptide used in the present invention or provided from heterologous sources by recombinant DNA techniques.

An "oligopeptide" is a short stretch of amino acid residues and may be expressed from an oligonucleotide. Oligopeptides comprise a stretch of amino acid residues of at least 3, 5, 10 amino acids and at most 10, 15, 25 amino acids, typically of at least 9 to 13 amino acids, and of sufficient length to display biological and/or antigenic activity.

"Inhibitor" is any substance which retards or prevents a chemical or physiological reaction or response. Common inhibitors include but are not limited to antisense molecules, antibodies, and antagonists.

"Standard expression" is a quantitative or qualitative measurement for comparison. It is based on a statistically appropriate number of normal samples and is created to use as a basis of comparison when performing diagnostic assays, running clinical trials, or following patient treatment profiles.

"Animal" as used herein may be defined to include human, domestic (e.g., cats, dogs, etc.), agricultural (e.g., cows, horses, sheep, etc.) or test species (e.g., mouse, rat, rabbit, etc.).

A "DRD3 polynucleotide", used in the invention, shall be understood as being a nucleic acid molecule selected from a group consisting of

(i) nucleic acid molecules encoding a polypeptide comprising the amino acid sequence of SEQ ID NO: 2,

(ii) nucleic acid molecules comprising the sequence of SEQ ID NO: 1,

(iii) nucleic acid molecules having the sequence of SEQ ID NO: 1,

(iv) nucleic acid molecules the complementary strand of which hybridizes under stringent conditions to a nucleic acid molecule of (i), (ii), or (iii); and

(v) nucleic acid molecules the sequence of which differs from the sequence of a nucleic, acid molecule of (iii) due to the degeneracy of the genetic code;

wherein the polypeptide encoded by said nucleic acid molecule has DRD3 activity.

A "DRD3 polypeptide", used in the invention, shall be understood as being a polypeptide selected from a group consisting of

(i) polypeptides having the sequence of SEQ ID NO: 2,

(ii) polypeptides comprising the sequence of SEQ ID NO: 2,

(iii) polypeptides encoded by DRD3 polynucleotides; and

(iv) polypeptides which show at least 99%, 98%, 95%, 90%, or 80% homology with a polypeptide of (i), (ii), or (iii);

wherein said polypeptide has DRD3 activity.

The nucleotide sequences encoding a DRD3 (or their complement) have numerous applications in techniques known to those skilled in the art of molecular biology. These techniques include use as hybridization probes, use in the construction of oligomers for PCR, use for chromosome and gene mapping, use in the recombinant production of DRD3, and use in generation of antisense DNA or RNA, their chemical analogs and the like. Uses of nucleotides encoding a DRD3 disclosed herein are exemplary of known techniques and are not intended to limit their use in any technique known to a person of ordinary skill in the art. Furthermore, the nucleotide sequences disclosed herein may be used in molecular biology techniques that have not yet been developed, provided the new techniques rely on properties of nucleotide sequences that are currently known, e.g., the triplet genetic code, specific base pair interactions, etc.

It will be appreciated by those skilled in the art that as a result of the degeneracy of the genetic code, a multitude of DRD3 - encoding nucleotide sequences may be produced. Some of these will only bear minimal homology to the nucleotide sequence of the known and naturally occurring DRD3. The disclosure has specifically contemplated each and every possible variation of nucleotide sequence that could be made by selecting combinations based on possible codon choices. These combinations are made in accordance with the standard triplet genetic code as applied to the nucleotide sequence of naturally occurring DRD3, and all such variations are to be considered as being specifically disclosed.

Although the nucleotide sequences which encode a DRD3, its derivatives or its variants are preferably capable of hybridizing to the nucleotide sequence of the naturally occurring DRD3 polynucleotide under stringent conditions, it may be advantageous to produce nucleotide sequences encoding DRD3 polypeptides or its derivatives possessing a substantially different codon usage. Codons can be selected to increase the rate at which expression of the peptide occurs in a particular prokaryotic or eukaryotic expression host in accordance with the frequency with which particular codons are utilized by the host. Other reasons for substantially altering the nucleotide sequence encoding a DRD3 polypeptide and/or its derivatives without altering the encoded amino acid sequence include the production of RNA transcripts having more desirable properties, such as a greater half-life, than transcripts produced from the naturally occurring sequence.

Nucleotide sequences encoding a DRD3 polypeptide may be joined to a variety of other nucleotide sequences by means of well established recombinant DNA techniques. Useful nucleotide sequences for joining to DRD3 polynucleotides include an assortment of cloning vectors such as plasmids, cosmids, lambda phage derivatives, phagemids, and the like. Vectors of interest include expression vectors, replication vectors, probe generation vectors, sequencing vectors, etc. In general, vectors of interest may contain an origin of replication functional in at least one organism, convenient restriction endonuclease sensitive sites, and selectable markers for one or more host cell systems.

Another aspect of the subject is to provide for DRD3-specific hybridization probes capable of hybridizing with naturally occurring nucleotide sequences encoding DRD3. Such probes may also be used for the detection of similar GPCR encoding sequences and should preferably show at least 40% nucleotide identity to DRD3 polynucleotides. The hybridization probes of the subject may be derived from the nucleotide sequence presented as SEQ ID NO: 1 or from genomic sequences including promoter, enhancers or introns of the native gene. Hybridization probes may be labelled by a variety of reporter molecules using techniques well known in the art.

It will be recognized that many deletional or mutational analogs of DRD3 polynucleotides will be effective hybridization probes for DRD3 polynucleotides. Accordingly, the disclosure relates to nucleic acid sequences that hybridize with such DRD3 encoding nucleic acid sequences under stringent conditions.

"Stringent conditions" refers to conditions that allow for the hybridization of substantially related nucleic acid sequences. For instance, such conditions will generally allow hybridization of sequence with at least about 85% sequence identity, preferably with at least about 90% sequence identity, more preferably with at least about 95% sequence identity. Hybridization conditions and probes can be adjusted in well-characterized ways to achieve selective hybridization of human-derived probes. Stringent conditions, within the meaning of the invention are 65°C in a buffer containing 1 mM EDTA, 0.5 M $NaHPO_4$ (pH 7.2), 7 % (w/v) SDS.

Nucleic acid molecules that will hybridize to DRD3 polynucleotides under stringent conditions can be identified functionally. Without limitation, examples of the uses for hybridization probes include: histochemical uses such as identifying tissues that express DRD3; measuring mRNA levels, for instance to identify a sample's tissue type or to identify cells that express abnormal levels of DRD3; and detecting polymorphisms of DRD3.

PCR provides additional uses for oligonucleotides based upon the nucleotide sequence which encodes DRD3. Such probes used in PCR may be of recombinant origin, chemically synthesized, or a mixture of both. Oligomers may comprise discrete nucleotide sequences employed under optimized conditions for identification of DRD3 in specific tissues or diagnostic use. The same two oligomers, a nested set of oligomers, or even a degenerate pool of oligomers may be employed under less stringent conditions for identification of closely related DNAs or RNAs.

Rules for designing polymerase chain reaction (PCR) primers are now established, as reviewed by PCR Protocols. Degenerate primers, i.e., preparations of primers that are heterogeneous at given sequence locations, can be designed to amplify nucleic acid sequences that are highly homologous to, but not identical with DRD3. Strategies are now available that allow for only one of the primers to be required to specifically hybridize with a known sequence. For example, appropriate nucleic acid primers can be ligated to the nucleic acid sought to be amplified to provide the hybridization partner for one of the primers. In this way, only one of the primers need be based on the sequence of the nucleic acid sought to be amplified.

PCR methods for amplifying nucleic acid will utilize at least two primers. One of these primers will be capable of hybridizing to a first strand of the nucleic acid to be amplified and of priming enzyme-driven nucleic acid synthesis in a first direction. The other will be capable of hybridizing the reciprocal sequence of the first strand (if the sequence to be amplified is single stranded, this sequence will initially be hypothetical, but will be synthesized in the first amplification cycle) and of priming nucleic acid synthesis from that strand in the direction opposite the first direction and towards the site of hybridization for the first primer. Conditions for conducting such amplifications, particularly under preferred stringent hybridization conditions, are well known.

Other means of producing specific hybridization probes for DRD3 include the cloning of nucleic acid sequences encoding DRD3 or DRD3 derivatives into vectors for the production of mRNA probes. Such vectors are known in the art, are commercially available and may be used to synthesize RNA probes in vitro by means of the addition of the appropriate RNA polymerase as T7 or SP6 RNA polymerase and the appropriate reporter molecules.

It is possible to produce a DNA sequence, or portions thereof, entirely by synthetic chemistry. After synthesis, the nucleic acid sequence can be inserted into any of the many available DNA vectors and their respective host cells using techniques which are well known in the art. Moreover, synthetic chemistry may be used to introduce mutations into the nucleotide sequence. Alternately, a portion of sequence in which a mutation is desired can be synthesized and recombined with longer portion of an existing genomic or recombinant sequence.

DRD3 polynucleotides may be used to produce a purified oligo-or polypeptide using well known methods of recombinant DNA technology. The oligopeptide may be expressed in a variety of host cells, either prokaryotic or eukaryotic. Host cells may be from the same species from which the nucleotide sequence was derived or from a different species. Advantages of producing an oligonucleotide by recombinant DNA technology include obtaining adequate amounts of the protein for purification and the availability of simplified purification procedures.

**Quantitative determinations of nucleic acids**

An important step in the molecular genetic analysis of human disease is often the enumeration of the copy number of a nucleis acid or the relative expression of a gene in particular tissues.

Several different approaches are currently available to make quantitative determinations of nucleic acids. Chromosome-based techniques, such as comparative genomic hybridization (CGH) and fluorescent in situ hybridization (FISH) facilitate efforts to cytogenetically localize genomic regions that are altered in tumor cells. Regions of genomic alteration can be narrowed further using loss of heterozygosity analysis (LOH), in which disease DNA is analyzed and compared with normal DNA for the loss of a heterozygous polymorphic marker. The first experiments used restriction fragment length polymorphisms (RFLPs) [Johnson, (1989)], or hypervariable minisatellite DNA [Barnes, 2000]. In recent years LOH has been performed primarily using PCR amplification of microsatellite markers and electrophoresis of the radio labelled [Jeffreys, (1985)] or fluorescently labelled PCR products [Weber, (1990)] and compared between paired normal and disease DNAs.

A number of other methods have also been developed to quantify nucleic acids [Gergen, (1992)]. More recently, PCR and RT-PCR methods have been developed which are capable of measuring the amount of a nucleic acid in a sample. One approach, for example, measures PCR product quantity in the log phase of the reaction before the formation of reaction products plateaus [Thomas, (1980)].

A gene sequence contained in all samples at relatively constant quantity is typically utilized for sample amplification efficiency normalization. This approach, however, suffers from several drawbacks. The method requires that each sample has equal input amounts of the nucleic acid and that the amplification efficiency between samples is identical until the time of analysis. Furthermore, it is difficult using the conventional methods of PCR quantitation such as gel electrophoresis or plate capture hybridization to determine that all samples are in fact analyzed during the log phase of the reaction as required by the method.

Another method called quantitative competitive (QC)-PCR, as the name implies, relies on the inclusion of an internal control competitor in each reaction [Piatak, (1993), BioTechniques]. The efficiency of each reaction is normalized to the internal competitor. A known amount of internal competitor is typically added to each sample. The unknown target PCR product is compared with the known competitor PCR product to obtain relative quantitation. A difficulty with this general approach lies in developing an internal control that amplifies with the same efficiency than the target molecule.

*5' Fluorogenic Nuclease Assays*

Fluorogenic nuclease assays are a real time quantitation method that uses a probe to monitor formation of amplification product. The basis for this method of monitoring the formation of amplification product is to measure continuously PCR product accumulation using a dual-labelled fluorogenic oligonucleotide probe, an approach frequently referred to in the literature simply as the "TaqMan method" [Piatak,(1993), Science; Heid, (1996); Gibson, (1996); Holland. (1991)].

The probe used in such assays is typically a short (about 20-25 bases) oligonucleotide that is labeled with two different fluorescent dyes. The 5' terminus of the probe is attached to a reporter dye and the 3' terminus is attached to a quenching dye, although the dyes could be attached at other locations on the probe as well. The probe is designed to have at least substantial sequence complementarity with the probe binding site. Upstream and downstream PCR primers which bind to flanking regions of the locus are added to the reaction mixture. When the probe is intact, energy transfer between the two fluorophors occurs and the quencher quenches emission from the reporter. During the extension phase of PCR, the probe is cleaved by the 5' nuclease activity of a nucleic acid polymerase such as Taq polymerase, thereby releasing the reporter from the oligonucleotide-quencher and resulting in an increase of reporter emission intensity which can be measured by an appropriate detector.

One detector which is specifically adapted for measuring fluorescence emissions such as those created during a fluorogenic assay is the ABI 7700 or 4700 HT manufactured by Applied Biosystems, Inc. in Foster City, Calif. The ABI 7700 uses fiber optics connected with each well in a 96-or 384 well PCR tube arrangement. The instrument includes a laser for exciting the labels and is capable of measuring the fluorescence spectra intensity from each tube with continuous monitoring during PCR amplification. Each tube is re-examined every 8.5 seconds.

Computer software provided with the instrument is capable of recording the fluorescence intensity of reporter and quencher over the course of the amplification. The recorded values will then be used to calculate the increase in normalized reporter emission intensity on a continuous basis. The increase in emission intensity is plotted versus time, i.e., the number of amplification cycles, to produce a continuous measure of amplification. To quantify the locus in each amplification reaction, the amplification plot is examined at a point during the log phase of product accumulation. This is accomplished by assigning a fluorescence threshold intensity above background and determining the point at which each amplification plot crosses the threshold (defined as the threshold cycle number or Ct). Differences in threshold cycle number are used to quantify the relative amount of PCR target contained within each tube. Assuming that each reaction functions at 100% PCR efficiency, a difference of one Ct represents a two-fold difference in the amount of starting template. The fluorescence value can be used in conjunction with a standard curve to determine the amount of amplification product present.

*Non-Probe-Based Detection Methods*

A variety of options are available for measuring the amplification products as they are formed. One method utilizes labels, such as dyes, which only bind to double stranded DNA. In this type of approach, amplification product (which is double stranded) binds dye molecules in solution to form a complex. With the appropriate dyes, it is possible to distinguish between dye molecules free in solution and dye molecules bound to amplification product. For example, certain dyes fluoresce only when bound to amplification product. Examples of dyes which can be used in methods of this general type include, but are not limited to, Syber Green.TM. and Pico Green from Molecular Probes, Inc. of Eugene, Oreg., ethidium bromide, propidium iodide, chromomycin, acridine orange, Hoechst 33258, Toto-1, Yoyo-1, DAPI (4',6-diamidino-2-phenylindole hydrochloride).

Another real time detection technique measures alteration in energy fluorescence energy transfer between fluorophors conjugated with PCR primers [Livak, (1995)].

*Probe-Based Detection Methods*

These detection methods involve some alteration to the structure or conformation of a probe hybridized to the locus between the amplification primer pair. In some instances, the alteration is caused by the template-dependent extension catalyzed by a nucleic acid polymerase during the amplification process. The alteration generates a detectable signal which is an indirect measure of the amount of amplification product formed.

For example, some methods involve the degradation or digestion of the probe during the extension reaction. These methods are a consequence of the 5'-3' nuclease activity associated with some nucleic acid polymerases. Polymerases having this activity cleave mononucleotides or small oligonucleotides from an oligonucleotide probe annealed to its complementary sequence located within the locus.

The 3' end of the upstream primer provides the initial binding site for the nucleic acid polymerase. As the polymerase catalyzes extension of the upstream primer and encounters the bound probe, the nucleic acid polymerase displaces a portion of the 5' end of the probe and through its nuclease activity cleaves mononucleotides or oligonucleotides from the probe.

The upstream primer and the probe can be designed such that they anneal to the complementary strand in close proximity to one another. In fact, the 3' end of the upstream primer and the 5' end of the probe may abut one another. In this situation, extension of the upstream primer is not necessary in order for the nucleic acid polymerase to begin cleaving the probe. In the case in which intervening nucleotides separate the upstream primer and the probe, extension of the primer is necessary before the nucleic acid polymerase encounters the 5' end of the probe. Once contact occurs and polymerization continues, the 5'-3' exonuclease activity of the nucleic acid polymerase begins cleaving mononucleotides or oligonucleotides from the 5' end of the probe. Digestion of the probe continues until the remaining portion of the probe dissociates from the complementary strand.

In solution, the two end sections can hybridize with each other to form a hairpin loop. In this conformation, the reporter and quencher dye are in sufficiently close proximity that fluorescence from the reporter dye is effectively quenched by the quencher dye. Hybridized probe, in contrast, results in a linearized conformation in which the extent of quenching is decreased. Thus, by monitoring emission changes for the two dyes, it is possible to indirectly monitor the formation of amplification product.

*Probes*

The labeled probe is selected so that its sequence is substantially complementary to a segment of the test locus or a reference locus. As indicated above, the nucleic acid site to which the probe binds should be located between the primer binding sites for the upstream and downstream amplification primers.

*Primers*

The primers used in the amplification are selected so as to be capable of hybridizing to sequences at flanking regions of the locus being amplified. The primers are chosen to have at least substantial complementarity with the different strands of the nucleic acid being amplified. When a probe is utilized to detect the formation of amplification products, the primers are selected in such that they flank the probe, i.e. are located upstream and downstream of the probe.

The primer must have sufficient length so that it is capable of priming the synthesis of extension products in the presence of an agent for polymerization. The length and composition of the primer depends on many parameters, including, for example, the temperature at which the annealing reaction is conducted, proximity of the probe binding site to that of the primer, relative concentrations of the primer and probe and the particular nucleic acid composition of the probe. Typically the primer includes 15-30 nucleotides. However, the length of the primer may be more or less depending on the complexity of the primer binding site and the factors listed above.

*Labels for Probes and Primers*

The labels used for labeling the probes or primers used in the current invention and which can provide the signal corresponding to the quantity of amplification product can take a variety of forms. As indicated above with regard to the 5' fluorogenic nuclease method, a fluorescent signal is one signal which can be measured. However, measurements may also be made, for example, by monitoring radioactivity, colorimetry, absorption, magnetic parameters, or enzymatic activity. Thus, labels which can be employed include, but are not limited to, fluorophors, chromophores, radioactive isotopes, electron dense reagents, enzymes, and ligands having specific binding partners (e.g., biotin-avidin).

Monitoring changes in fluorescence is a particularly useful way to monitor the accumulation of amplification products. A number of labels useful for attachment to probes or primers are commercially available including fluorescein and various fluorescein derivatives such as FAM, HEX, TET and JOE (all which are available from Applied Biosystems, Foster City, Calif.); lucifer yellow, and coumarin derivatives.

Labels may be attached to the probe or primer using a variety of techniques and can be attached at the 5' end, and/or the 3' end and/or at an internal nucleotide. The label can also be attached to spacer arms of various sizes which are attached to the probe or primer. These spacer arms are useful for obtaining a desired distance between multiple labels attached to the probe or primer.

In some instances, a single label may be utilized; whereas, in other instances, such as with the 5' fluorogenic nuclease assays for example, two or more labels are attached to the probe. In cases wherein the probe includes multiple labels, it is generally advisable to maintain spacing between the labels which is sufficient to permit separation of the labels during digestion of the probe through the 5'-3' nuclease activity of the nucleic acid polymerase.

**Patients Exhibiting Symptoms of Disease**

A number of diseases are associated with changes in the copy number of a certain gene. For patients having symptoms of a disease, the real-time PCR method can be used to determine if the patient has copy number alterations which are known to be linked with diseases that are associated with the symptoms the patient has.

**DRD3 expression**

*DRD3 fusion proteins*

Fusion proteins are useful for generating antibodies against DRD3 polypeptides and for use in various assay systems. For example, fusion proteins can be used to identify proteins which interact with portions of DRD3 polypeptides. Protein affinity chromatography or library-based assays for protein-protein interactions, such as the yeast two-hybrid or phage display systems, can be used for this purpose. Such methods are well known in the art and also can be used as drug screens.

A DRD3 fusion protein comprises two polypeptide segments fused together by means of a peptide bond. The first polypeptide segment can comprise at least 54, 75, 100, 125, 139, 150, 175, 200, 225, 250, or 275 contiguous amino acids of SEQ ID NO: 2 or of a biologically active variant, such as those described above. The first polypeptide segment also can comprise full-length DRD3.

The second polypeptide segment can be a full-length protein or a protein fragment. Proteins commonly used in fusion protein construction include, but are not limited to β galactosidase, β-glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions, including histidine (His) tags, FLAG tags, influenza hemagglutinin (HA) tags, Myc tags, VSV-G tags, and thioredoxin (Trx) tags. Other fusion constructions can include maltose binding protein (MBP), S-tag, Lex a DNA binding domain (DBD) fusions, GAL4 DNA binding domain fusions, herpes simplex virus (HSV) BP16 protein fusions and G-protein fusions (for example G(alpha)16, Gs, Gi). A fusion protein also can be engineered to contain a cleavage site located adjacent to the DRD3.

*Preparation of Polynucleotides*

A naturally occurring DRD3 polynucleotide can be isolated free of other cellular components such as membrane components, proteins, and lipids. Polynucleotides can be made by a cell and isolated using standard nucleic acid purification techniques, or synthesized using an amplification technique, such as the polymerase chain reaction (PCR), or by using an automatic synthesizer. Methods for isolating polynucleotides are routine and are known in the art. Any such technique for obtaining a polynucleotide can be used to obtain isolated DRD3 polynucleotides. For example, restriction enzymes and probes can be used to isolate polynucleotide fragments which comprise DRD3 nucleotide sequences. Isolated polynucleotides are in preparations which are free or at least 70, 80, or 90% free of other molecules.

DRD3 cDNA molecules can be made with standard molecular biology techniques, using DRD3 mRNA as a template. DRD3 cDNA molecules can thereafter be replicated using molecular biology techniques known in the art. An amplification technique, such as PCR, can be used to obtain additional copies of polynucleotide used in the invention, using either human genomic DNA or cDNA as a template.

Alternatively, synthetic chemistry techniques can be used to synthesizes DRD3 polynucleotides. The degeneracy of the genetic code allows alternate nucleotide sequences to be synthesized which will encode DRD3 having, for example, an amino acid sequence shown in SEQ ID NO: 2 or a biologically active variant thereof.

*Extending Polynucleotides*

Various PCR-based methods can be used to extend nucleic acid sequences encoding human DRD3, for example to detect upstream sequences of DRD3 gene such as promoters and regulatory elements. For example, restriction-site PCR uses universal primers to retrieve unknown sequence adjacent to a known locus. Genomic DNA is first amplified in the presence of a primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Inverse PCR also can be used to amplify or extend sequences using divergent primers based on a known region. Primers can be designed using commercially available software, such as OLIGO 4.06 Primer Analysis software (National Biosciences Inc., Plymouth, Minn.), to be 22-30 nucleotides in length, to have a GC content of 50% or more, and to anneal to the target sequence at temperatures about 68-72°C. The method uses several restriction enzymes to generate a suitable fragment in the known region of a gene. The fragment is then circularized by intramolecular ligation and used as a PCR template.

Another method which can be used is capture PCR, which involves PCR amplification of DNA fragments adjacent to a known sequence in human and yeast artificial chromosome DNA. In this method, multiple restriction enzyme digestions and ligations also can be used to place an engineered double-stranded sequence into an unknown fragment of the DNA molecule before performing PCR.

When screening for full-length cDNAs, it is preferable to use libraries that have been size-selected to include larger cDNAs. Randomly-primed libraries are preferable, in that they will contain more sequences which contain the 5' regions of genes. Use of a randomly primed library may be especially preferable for situations in which an oligo d(T) library does not yield a full-length cDNA. Genomic libraries can be useful for extension of sequence into 5' non-transcribed regulatory regions.

Commercially available capillary electrophoresis systems can be used to analyze the size or confirm the nucleotide sequence of PCR or sequencing products. For example, capillary sequencing can employ flowable polymers for electrophoretic separation, four different fluorescent dyes (one for each nucleotide) which are laser activated, and detection of the emitted wavelengths by a charge coupled device camera. Output/light intensity can be converted to electrical signal using appropriate equipment and software (e.g., GENOTYPER and Sequence NAVIGATOR, Perkin Elmer), and the entire process from loading of samples to computer analysis and electronic data display can be computer controlled. Capillary electrophoresis is especially preferable for the sequencing of small pieces of DNA which might be present in limited amounts in a particular sample.

*Obtaining Polypeptides*

DRD3 can be obtained, for example, by purification from human cells, by expression of DRD3 polynucleotides, or by direct chemical synthesis.

*Protein Purification*

DRD3 can be purified from any human cell which expresses the receptor, including those which have been transfected with expression constructs which express DRD3. A purified DRD3 is separated from other compounds which normally associate with DRD3 in the cell, such as certain proteins, carbohydrates, or lipids, using methods well-known in the art. Such methods include, but are not limited to, size exclusion chromatography, ammonium sulfate fractionation, ion exchange chromatography, affinity chromatography, and preparative gel electrophoresis.

*Expression of DRD3 Polynucleotides*

To express DRD3, DRD3 polynucleotides can be inserted into an expression vector which contains the necessary elements for the transcription and translation of the inserted coding sequence. Methods which are well known to those skilled in the art can be used to construct expression vectors containing sequences encoding DRD3 and appropriate transcriptional and translational control elements. These methods include *in vitro* recombinant DNA techniques, synthetic techniques, and *in vivo* genetic recombination.

A variety of expression vector/host systems can be utilized to contain and express sequences encoding DRD3. These include, but are not limited to, microorganisms, such as bacteria transformed with recombinant bacteriophage, plasmid, or cosmid DNA expression vectors; yeast transformed with yeast expression vectors, insect cell systems infected with virus expression vectors (e.g., baculovirus), plant cell systems transformed with virus expression vectors (*e.g.*, cauliflower mosaic virus, CaMV; tobacco mosaic virus, TMV) or with bacterial expression vectors *(e.g.,* Ti or pBR322 plasmids), or animal cell systems.

The control elements or regulatory sequences are those non-translated regions of the vector - enhancers, promoters, 5' and 3' untranslated regions -- which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORT1 plasmid (Life Technologies) and the like can be used. The baculovirus polyhedrin promoter can be used in insect cells. Promoters or enhancers derived from the genomes of plant cells *(e.g.,* heat shock, RUBISCO, and storage protein genes) or from plant viruses (e.g., viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding DRD3, vectors based on SV40 or EBV can be used with an appropriate selectable marker.

*Bacterial and Yeast Expression Systems*

In bacterial systems, a number of expression vectors can be selected. For example, when a large quantity of DRD3 is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding DRD3 can be ligated into the vector in frame with sequences for the amino-terminal Met and the subsequent 7 residues of β-galactosidase so that a hybrid protein is produced. pIN vectors or pGEX vectors (Promega, Madison, Wis.) also can be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems can be designed to include heparin, thrombin, or factor Xa protease cleavage sites so that the cloned polypeptide of interest can be released from the GST moiety at will.

*Plant and Insect Expression Systems*

If plant expression vectors are used, the expression of sequences encoding DRD3 can be driven by any of a number of promoters. For example, viral promoters such as the 35S and 19S promoters of CaMV can be used alone or in combination with the omega leader sequence from TMV. Alternatively, plant promoters such as the small subunit of RUBISCO or heat shock promoters can be used. These constructs can be introduced into plant cells by direct DNA transformation or by pathogen-mediated transfection.

An insect system also can be used to express DRD3. For example, in one such system *Autographa californica* nuclear polyhedrosis virus (AcNPV) is used as a vector to express foreign genes in *Spodoptera frugiperda* cells or in *Trichoplusia* larvae. Sequences encoding DRD3 can be cloned into a non-essential region of the virus, such as the polyhedrin gene, and placed under control of the polyhedrin promoter. Successful insertion of DRD3 will render the polyhedrin gene inactive and produce recombinant virus lacking coat protein. The recombinant viruses can then be used to infect *S. frugiperda cells* or *Trichoplusia* larvae in which DRD3 can be expressed.

*Mammalian Expression Systems*

A number of viral-based expression systems can be used to express DRD3 in mammalian host cells. For example, if an adenovirus is used as an expression vector, sequences encoding DRD3 can be ligated into an adenovirus transcription/translation complex comprising the late promoter and tripartite leader sequence. Insertion in a non-essential E1 or E3 region of the viral genome can be used to obtain a viable virus which is capable of expressing DRD3 in infected host cells [Engelhard, 1994)]. If desired, transcription enhancers, such as the Rous sarcoma virus (RSV) enhancer, can be used to increase expression in mammalian host cells.

Human artificial chromosomes (HACs) also can be used to deliver larger fragments of DNA than can be contained and expressed in a plasmid. HACs of 6M to 10M are constructed and delivered to cells via conventional delivery methods (e.g., liposomes, polycationic amino polymers, or vesicles). Specific initiation signals also can be used to achieve more efficient translation of sequences encoding DRD3. Such signals include the ATG initiation codon and adjacent sequences. In cases where sequences encoding DRD3, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic.

*Host Cells*

A host cell strain can be chosen for its ability to modulate the expression of the inserted sequences or to process the expressed DRD3 in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for post-translational activities *(e.g.,* CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express DRD3 can be transformed using expression vectors which can contain viral origins of replication and/or endogenous expression elements and a selectable marker gene on the same or on a separate vector. Following the introduction of the vector, cells can be allowed to grow for 1-2 days in an enriched medium before they are switched to a selective medium. The purpose of the selectable marker is to confer resistance to selection, and its presence allows growth and recovery of cells which successfully express the introduced DRD3 sequences. Resistant clones of stably transformed cells can be proliferated using tissue culture techniques appropriate to the cell type. Any number of selection systems can be used to recover transformed cell lines. These include, but are not limited to, the herpes simplex virus thymidine kinase [Logan, (1984)] and adenine phosphoribosyltransferase [Wigler, (1977)] genes which can be employed in *tk*$^-$ or *aprt*$^-$ cells, respectively. Also, antimetabolite, antibiotic, or herbicide resistance can be used as the basis for selection. For example, *dhfr* confers resistance to methotrexate [Lowy, (1980)], *npt* confers resistance to the aminoglycosides, neomycin and G-418 [Wigler, (1980)], and *als* and *pat* confer resistance to chlorsulfuron and phosphinotricin acetyltransferase, respectively [Colbere-Garapin, 1981]. Additional selectable genes have been described. For example, *trpB* allows cells to utilize indole in place of tryptophan, or *hisD,* which allows cells to utilize histinol in place of histidine. Visible markers such as anthocyanins, β-glucuronidase and its substrate GUS, and luciferase and its substrate luciferin, can be used to identify transformants and to quantify the amount of transient or stable protein expression attributable to a specific vector system

*Detecting Polypeptide Expression*

Although the presence of marker gene expression suggests that a DRD3 polynucleotide is also present, its presence and expression may need to be confirmed. For example, if a sequence encoding DRD3 is inserted within a marker gene sequence, transformed cells containing sequences which encode DRD3 can be identified by the absence of marker gene function. Alternatively, a marker gene can be placed in tandem with a sequence encoding DRD3 under the control of a single promoter. Expression of the marker gene in response to induction or selection usually indicates expression of DRD3 polynucleotide.

Alternatively, host cells which contain a DRD3 polynucleotide and which express DRD3 can be identified by a variety of procedures known to those of skill in the art. These procedures include, but are not limited to, DNA-DNA or DNA-RNA hybridizations and protein bioassay or immunoassay techniques which include membrane, solution, or chip-based technologies for the detection and/or quantification of nucleic acid or protein. For example, the presence of a polynucleotide sequence encoding DRD3 can be detected by DNA-DNA or DNA-RNA hybridization or amplification using probes or fragments or fragments of polynucleotides encoding DRD3. Nucleic acid amplification-based assays involve the use of oligonucleotides selected from sequences encoding DRD3 to detect transformants which contain a DRD3 polynucleotide.

A variety of protocols for detecting and measuring the expression of DRD3, using either polyclonal or monoclonal antibodies specific for the polypeptide, are known in the art. Examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on DRD3 can be used, or a competitive binding assay can be employed.

A wide variety of labels and conjugation techniques are known by those skilled in the art and can be used in various nucleic acid and amino acid assays. Means for producing labeled hybridization or PCR probes for detecting sequences related to polynucleotides encoding DRD3 include oligolabeling, nick translation, end-labeling, or PCR amplification using a labeled nucleotide. Alternatively, sequences encoding DRD3 can be cloned into a vector for the production of an mRNA probe. Such vectors are known in the art, are commercially available, and can be used to synthesize RNA probes *in vitro* by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

*Expression and Purification of Polypeptides*

Host cells transformed with DRD3 polynucleotides can be cultured under conditions suitable for the expression and recovery of the protein from cell culture. The polypeptide produced by a transformed cell can be secreted or contained intracellularly depending on the sequence and/or the vector used. As will be understood by those of skill in the art, expression vectors containing DRD3 polynucleotides can be designed to contain signal sequences which direct secretion of soluble DRD3 through a prokaryotic or eukaryotic cell membrane or which direct the membrane insertion of membrane-bound DRD3.

As discussed above, other constructions can be used to join a sequence encoding DRD3 to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor XA or enterokinase (Invitrogen, San Diego, CA) between the purification domain and DRD3 also can be used to facilitate purification. One such expression vector provides for expression of a fusion protein containing DRD3 and 6 histidine residues preceding a thioredoxin or an enterokinase cleavage site. The histidine residues facilitate purification by IMAC (immobilized metal ion affinity chromatography) Maddox, (1983)], while the enterokinase cleavage site provides a means for purifying DRD3 from the fusion protein [Porath, (1992)].

*Chemical Synthesis*

Sequences encoding DRD3 can be synthesized, in whole or in part, using chemical methods well known in the art. Alternatively, DRD3 itself can be produced using chemical methods to synthesize its amino acid sequence, such as by direct peptide synthesis using solid-phase techniques. Protein synthesis can either be performed using manual techniques or by automation. Automated synthesis can be achieved, for example, using Applied Biosystems 431A Peptide Synthesizer (Perkin Elmer). Optionally, fragments of DRD3 can be separately synthesized and combined using chemical methods to produce a full-length molecule.

The newly synthesized peptide can be substantially purified by preparative high performance liquid chromatography. The composition of a synthetic DRD3 can be confirmed by amino acid analysis or sequencing. Additionally, any portion of the amino acid sequence of DRD3 can be altered during direct synthesis and/or combined using chemical methods with sequences from other proteins to produce a variant polypeptide or a fusion protein.

*Production of Altered Polypeptides*

As will be understood by those of skill in the art, it may be advantageous to produce DRD3 polynucleotides possessing non-naturally occurring codons. For example, codons preferred by a particular prokaryotic or eukaryotic host can be selected to increase the rate of protein expression or to produce an RNA transcript having desirable properties, such as a half-life which is longer than that of a transcript generated from the naturally occurring sequence.
The nucleotide sequences referred to herein can be engineered using methods generally known in the art to alter DRD3 polynucleotides for a variety of reasons, including but not limited to, alterations which modify the cloning, processing, and/or expression of the polypeptide or mRNA product. DNA shuffling by random fragmentation and PCR reassembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

*Antibodies*

Any type of antibody known in the art can be generated to bind specifically to an epitope of DRD3.
"Antibody" as used herein includes intact immunoglobulin molecules, as well as fragments thereof, such as Fab, $F(ab')_2$, and Fv, which are capable of binding an epitope of DRD3. Typically, at least 6, 8, 10, or 12 contiguous amino acids are required to form an epitope. However, epitopes which involve non-contiguous amino acids may require more, e.g., at least 15, 25, or 50 amino acid. An antibody which specifically binds to an epitope of DRD3 can be used therapeutically, as well as in immunochemical assays, such as Western blots, ELISAs, radioimmunoassays, immunohistochemical assays, immunoprecipitations, or other immunochemical assays known in the art. Various immunoassays can be used to identify antibodies having the desired specificity. Numerous protocols for competitive binding or immunoradiometric assays are well known in the art. Such immunoassays typically involve the measurement of complex formation between an immunogen and an antibody which specifically binds to the DRD3 immunogen.
Typically, an antibody which specifically binds to DRD3 provides a detection signal at least 5-, 10-, or 20-fold higher than a detection signal provided with other proteins when used in an immunochemical assay. Preferably, antibodies which specifically bind to DRD3 do not detect other proteins in immunochemical assays and can immunoprecipitate DRD3 from solution.
DRD3 can be used to immunize a mammal, such as a mouse, rat, rabbit, guinea pig, monkey, or human, to produce polyclonal antibodies. If desired, DRD3 can be conjugated to a carrier protein, such as bovine serum albumin, thyroglobulin, and keyhole limpet hemocyanin. Depending on the host species, various adjuvants can be used to increase the immunological response. Such adjuvants include, but are not limited to, Freund's adjuvant, mineral gels (e.g., aluminum hydroxide), and surface active substances (e.g., lysolecithin, pluronic polyols, polyanions, peptides, oil emulsions, keyhole limpet hemocyanin, and dinitrophenol). Among adjuvants used in humans, BCG *(bacilli Calmette-Guerin)* and *Corynebacterium parvum* are especially useful.
Monoclonal antibodies which specifically bind to DRD3 can be prepared using any technique which provides for the production of antibody molecules by continuous cell lines in culture. These techniques include, but are not limited to, the hybridoma technique, the human B-cell hybridoma technique, and the EBV-hybridoma technique [Roberge, (1995)].
In addition, techniques developed for the production of "chimeric antibodies", the splicing of mouse antibody genes to human antibody genes to obtain a molecule with appropriate antigen specificity and biological activity, can be used. Monoclonal and other antibodies also can be "humanized" to prevent a patient from mounting an immune response against the antibody when it is used therapeutically. Such antibodies may be sufficiently similar in sequence to human antibodies to be used directly in therapy or may require alteration of a few key residues. Sequence differences between rodent antibodies and human sequences can be minimized by replacing residues which differ from those in the human sequences by site directed mutagenesis of individual residues or by grating of entire complementarity determining regions. Antibodies which specifically bind to DRD3 can contain antigen binding sites which are either partially or fully humanized, as disclosed in U.S. 5,565,332.
Alternatively, techniques described for the production of single chain antibodies can be adapted using methods known in the art to produce single chain antibodies which specifically bind to DRD3. Antibodies with related specificity, but of distinct idiotypic composition, can be generated by chain shuffling from random combinatorial immunoglobin libraries. Single-chain antibodies also can be constructed using a DNA amplification method, such as PCR, using hybridoma cDNA as a template. Single-chain antibodies can be mono- or bispecific, and can be bivalent or tetravalent. Construction of tetravalent, bispecific single-chain antibodies is taught. A nucleotide sequence encoding a single-chain antibody can be constructed using manual or automated nucleotide synthesis, cloned into an expression construct using standard recombinant DNA methods, and introduced into a cell to express the coding sequence, as described below. Alternatively, single-chain antibodies can be produced directly using, for example, filamentous phage technology.

Antibodies which specifically bind to DRD3 also can be produced by inducing *in vivo* production in the lymphocyte population or by screening immunoglobulin libraries or panels of highly specific binding reagents. Other types of antibodies can be constructed and used therapeutically in methods of the invention. For example, chimeric antibodies can be constructed as disclosed in WO 93/03151. Binding proteins which are derived from immunoglobulins and which are multivalent and multispecific, such as the "diabodies" described in WO 94/13804, also can be prepared.

Antibodies according to the invention can be purified by methods well known in the art. For example, antibodies can be affinity purified by passage over a column to which DRD3 is bound. The bound antibodies can then be eluted from the column using a buffer with a high salt concentration.

*Antisense Oligonucleotides*

Antisense oligonucleotides are nucleotide sequences which are complementary to a specific DNA or RNA sequence. Once introduced into a cell, the complementary nucleotides combine with natural sequences produced by the cell to form complexes and block either transcription or translation. Preferably, an antisense oligonucleotide is at least 11 nucleotides in length, but can be at least 12, 15, 20, 25, 30, 35, 40, 45, or 50 or more nucleotides long. Longer sequences also can be used. Antisense oligonucleotide molecules can be provided in a DNA construct and introduced into a cell as described above to decrease the level of DRD3 gene products in the cell.

Antisense oligonucleotides can be deoxyribonucleotides, ribonucleotides, or a combination of both. Oligonucleotides can be synthesized manually or by an automated synthesizer, by covalently linking the 5' end of one nucleotide with the 3' end of another nucleotide with non-phosphodiester internucleotide linkages such alkylphosphonates, phosphorothioates, phosphorodithioates, alkylphosphonothioates, alkylphosphonates, phosphoramidates, phosphate esters, carbamates, acetamidate, carboxymethyl esters, carbonates, and phosphate triesters.

Modifications of DRD3 gene expression can be obtained by designing antisense oligonucleotides which will form duplexes to the control, 5', or regulatory regions of the DRD3 gene. Oligonucleotides derived from the transcription initiation site, e.g., between positions -10 and +10 from the start site, are preferred. Similarly, inhibition can be achieved using "triple helix" base-pairing methodology. Triple helix pairing is useful because it causes inhibition of the ability of the double helix to open sufficiently for the binding of polymerases, transcription factors, or chaperons. Therapeutic advances using triplex DNA have been described in the literature [Nicholls, (1993)]. An antisense oligonucleotide also can be designed to block translation of mRNA by preventing the transcript from binding to ribosomes.

Precise complementarity is not required for successful complex formation between an antisense oligonucleotide and the complementary sequence of a DRD3 polynucleotide. Antisense oligonucleotides which comprise, for example, 2, 3, 4, or 5 or more stretches of contiguous nucleotides which are precisely complementary to a DRD3 polynucleotide, each separated by a stretch of contiguous nucleotides which are not complementary to adjacent DRD3 nucleotides, can provide sufficient targeting specificity for DRD3 mRNA. Preferably, each stretch of complementary contiguous nucleotides is at least 4, 5, 6, 7, or 8 or more nucleotides in length. Non-complementary intervening sequences are preferably 1, 2, 3, or 4 nucleotides in length. One skilled in the art can easily use the calculated melting point of an antisense-sense pair to determine the degree of mismatching which will be tolerated between a particular antisense oligonucleotide and a particular DRD3 polynucleotide sequence. Antisense oligonucleotides can be modified without affecting their ability to hybridize to a DRD3 polynucleotide. These modifications can be internal or at one or both ends of the antisense molecule. For example, internucleoside phosphate linkages can be modified by adding cholesteryl or diamine moieties with varying numbers of carbon residues between the amino groups and terminal ribose. Modified bases and/or sugars, such as arabinose instead of ribose, or a 3', 5'-substituted oligonucleotide in which the 3' hydroxyl group or the 5' phosphate group are substituted, also can be employed in a modified antisense oligonucleotide. These modified oligonucleotides can be prepared by methods well known in the art.

*Ribozymes*

Ribozymes are RNA molecules with catalytic activity [Uhlmann, (1987)]. Ribozymes can be used to inhibit gene function by cleaving an RNA sequence, as is known in the art. The mechanism of ribozyme action involves sequence-specific hybridization of the ribozyme molecule to complementary target RNA, followed by endonucleolytic cleavage. Examples include engineered hammerhead motif ribozyme molecules that can specifically and efficiently catalyze endonucleolytic cleavage of specific nucleotide sequences. The coding sequence of a DRD3 polynucleotide can be used to generate ribozymes which will specifically bind to mRNA transcribed from a DRD3 polynucleotide. Methods of designing and constructing ribozymes which can cleave other RNA molecules in trans in a highly sequence specific manner have been developed and described in the art. For example, the cleavage activity of ribozymes can be targeted to specific RNAs by engineering a discrete "hybridization" region into the ribozyme. The hybridization region contains a sequence complementary to the target RNA and thus specifically hybridizes with the target RNA.

Specific ribozyme cleavage sites within a DRD3 RNA target can be identified by scanning the target molecule for ribozyme cleavage sites which include the following sequences: GUA, GUU, and GUC. Once identified, short RNA sequences of between 15 and 20 ribonucleotides corresponding to the region of the target RNA containing the cleavage site can be evaluated for secondary structural features which may render the target inoperable. Suitability of candidate DRD3 RNA targets also can be evaluated by testing accessibility to hybridization with complementary oligonucleotides using ribonuclease protection assays. The nucleotide sequences shown in SEQ ID NO: 1 and its complement provide sources of suitable hybridization region sequences. Longer complementary sequences can be used to increase the affinity of the hybridization sequence for the target. The hybridizing and cleavage regions of the ribozyme can be integrally related such that upon hybridizing to the target RNA through the complementary regions, the catalytic region of the ribozyme can cleave the target.

Ribozymes can be introduced into cells as part of a DNA construct. Mechanical methods, such as microinjection, liposome-mediated transfection, electroporation, or calcium phosphate precipitation, can be used to introduce a ribozyme-containing DNA construct into cells in which it is desired to decrease DRD3 expression. Alternatively, if it is desired that the cells stably retain the DNA construct, the construct can be supplied on a plasmid and maintained as a separate element or integrated into the genome of the cells, as is known in the art. A ribozyme-encoding DNA construct can include transcriptional regulatory elements, such as a promoter element, an enhancer or UAS element, and a transcriptional terminator signal, for controlling transcription of ribozymes in the cells (U.S. 5,641,673). Ribozymes also can be engineered to provide an additional level of regulation, so that destruction of mRNA occurs only when both a ribozyme and a target gene are induced in the cells.

**Screening / Screening Assays**

*Regulators*

Regulators as used herein, refer to compounds that affect the activity of a DRD3 in vivo and/or in vivo. Regulators can be agonists and antagonists of a DRD3 polypeptide and can be compounds that exert their effect on the DRD3 activity via the expression, via post-translational modifications or by other means. Agonists of DRD3 are molecules which, when bound to DRD3, increase or prolong the activity of DRD3. Agonists of DRD3 include proteins, nucleic acids, carbohydrates, small molecules, or any other molecule which activate DRD3. Antagonists of DRD3 are molecules which, when bound to DRD3, decrease the amount or the duration of the activity of DRD3. Antagonists include proteins, nucleic acids, carbohydrates, antibodies, small molecules, or any other molecule which decrease the activity of DRD3.

The term "modulate", as it appears herein, refers to a change in the activity of DRD3 polypeptide. For example, modulation may cause an increase or a decrease in protein activity, binding characteristics, or any other biological, functional, or immunological properties of DRD3.

As used herein, the terms "specific binding" or "specifically binding" refer to that interaction between a protein or peptide and an agonist, an antibody, or an antagonist. The interaction is dependent upon the presence of a particular structure of the protein recognized by the binding molecule (i.e., the antigenic determinant or epitope). For example, if an antibody is specific for epitope "A" the presence of a polypeptide containing the epitope A, or the presence of free unlabeled A, in a reaction containing free labeled A and the antibody will reduce the amount of labeled A that binds to the antibody.

The invention provides methods (also referred to herein as "screening assays") for identifying compounds which can be used for the treatment of heart failure, ischemic diseases of the heart, myocardial infarction and peripheral vascular diseases

The methods entail the identification of candidate or test compounds or agents (e.g., peptides, peptidomimetics, small molecules or other molecules) which bind to DRD3 and/or have a stimulatory or inhibitory effect on the biological activity of DRD3 or its expression and then determining which of these compounds have an effect on symptoms or diseases regarding heart failure, ischemic diseases of the heart, myocardial infarction and peripheral vascular diseases in an *in vivo* assay.

Candidate or test compounds or agents which bind to DRD3 and/or have a stimulatory or.inhibitory effect on the activity or the expression of DRD3 are identified either in assays that employ cells which express DRD3 on the cell surface (cell-based assays) or in assays with isolated DRD3 (cell-free assays). The various assays can employ a variety of variants of DRD3 (e.g., full-length DRD3, a biologically active fragment of DRD3, or a fusion protein which includes all or a portion of DRD3). Moreover, DRD3 can be derived from any suitable mammalian species (e.g., human DRD3, rat DRD3 or murine DRD3). The assay can be a binding assay entailing direct or indirect measurement of the binding of a test compound or a known DRD3 ligand to DRD3. The assay can also be an activity assay entailing direct or indirect measurement of the activity of DRD3. The assay can also be an expression assay entailing direct or indirect measurement of the expression of DRD3 mRNA or DRD3 protein. The various screening assays are combined with an *in vivo* assay entailing measuring the effect of the test compound on the symptoms of heart failure, ischemic diseases of the heart, myocardial infarction and peripheral vascular diseases

In one embodiment, the invention provides assays for screening candidate or test compounds which bind to or modulate the activity of a membrane-bound (cell surface expressed) form of DRD3. Such assays can employ full-length DRD3, a biologically active fragment of DRD3, or a fusion protein which includes all or a portion of DRD3. As described in greater detail below, the test compound can be obtained by any suitable means, e.g., from conventional compound libraries. Determining the ability of the test compound to bind to a membrane-bound form of DRD3 can be accomplished, for example, by coupling the test compound with a radioisotope or enzymatic label such that binding of the test compound to the DRD3 -expressing cell can be measured by detecting the labeled compound in a complex. For example, the test compound can be labelled with $^{125}I$, $^{35}S$, $^{14}C$, or $^{3}H$, either directly or indirectly, and the radioisotope detected by direct counting of radioemmission or by scintillation counting. Alternatively, the test compound can be enzymatically labelled with, for example, horseradish peroxidase, alkaline phosphatase, or luciferase, and the enzymatic label detected by determination of conversion of an appropriate substrate to product.

In a competitive binding format, the assay comprises contacting DRD3 expressing cell with a known compound which binds to DRD3 to form an assay mixture, contacting the assay mixture with a test compound, and determining the ability of the test compound to interact with the DRD3 expressing cell, wherein determining the ability of the test compound to interact with the DRD3 expressing cell comprises determining the ability of the test compound to preferentially bind the DRD3 expressing cell as compared to the known compound.

In another embodiment, the assay is a cell-based assay comprising contacting a cell expressing a membrane-bound form of DRD3 (e.g., full-length DRD3, a biologically active fragment of DRD3, or a fusion protein which includes all or a portion of DRD3) expressed on the cell surface with a test compound and determining the ability of the test compound to modulate (e.g., stimulate or inhibit) the activity of the membrane-bound form of DRD3. Determining the ability of the test compound to modulate the activity of the membrane-bound form of DRD3 can be accomplished by any method suitable for measuring the activity of DRD3, e.g., any method suitable for measuring the activity of a G-protein coupled receptor or other seven-transmembrane receptor (described in greater detail below). The activity of a seven-transmembrane receptor can be measured in a number of ways, not all of which are suitable for any given receptor. Among the measures of activity are: alteration in intracellular $Ca^{2+}$ concentration, activation of phospholipase C, alteration in intracellular inositol triphosphate ($IP_3$) concentration, alteration in intracellular diacylglycerol (DAG) concentration, and alteration in intracellular adenosine cyclic 3', 5'-monophosphate (cAMP) concentration.

Determining the ability of the test compound to modulate the activity of DRD3 can be accomplished, for example, by determining the ability of DRD3 to bind to or interact with a target molecule. The target molecule can be a molecule with which DRD3 binds or interacts with in nature, for example, a molecule on the surface of a cell which expresses DRD3, a molecule on the surface of a second cell, a molecule in the extracellular milieu, a molecule associated with the internal surface of a cell membrane or a cytoplasmic molecule. The target molecule can be a component of a signal transduction pathway which facilitates transduction of an extracellular signal (e.g., a signal generated by binding of a DRD3 ligand, through the cell membrane and into the cell. The target DRD3 molecule can be, for example, a second intracellular protein which has catalytic activity or a protein which facilitates the association of downstream signaling molecules with DRD3.

Determining the ability of DRD3 to bind to or interact with a target molecule can be accomplished by one of the methods described above for determining direct binding. In one embodiment, determining the ability of a polypeptide of the invention to bind to or interact with a target molecule can be accomplished by determining the activity of the target molecule. For example, the activity of the target molecule can be determined by detecting induction of a cellular second messenger of the target (e.g., intracellular $Ca^{2+}$, diacylglycerol, $IP_3$, etc.), detecting catalytic/enzymatic activity of the target on an appropriate substrate, detecting the induction of a reporter gene (e.g., a regulatory element that is responsive to a polypeptide of the invention operably linked to a nucleic acid encoding a detectable marker, e.g., luciferase), or detecting a cellular response.

The present invention also includes cell-free assays. Such assays involve contacting a form of DRD3 (e.g., full-length DRD3, a biologically active fragment of DRD3, or a fusion protein comprising all or a portion of DRD3) with a test compound and determining the ability of the test compound to bind to DRD3. Binding of the test compound to DRD3 can be determined either directly or indirectly as described above. In one embodiment, the assay includes contacting DRD3 with a known compound which binds DRD3 to form an assay mixture, contacting the assay mixture with a test compound, and determining the ability of the test compound to interact with DRD3, wherein determining the ability of the test compound to interact with DRD3 comprises determining the ability of the test compound to preferentially bind to DRD3 as compared to the known compound.

The cell-free assays of the present invention are amenable to use of either a membrane-bound form of DRD3 or a soluble fragment thereof. In the case of cell-free assays comprising the membrane-bound form of the polypeptide, it may be desirable to utilize a solubilizing agent such that the membrane-bound form of the polypeptide is maintained in solution. Examples of such solubilizing agents include but are not limited to non-ionic detergents such as n-octylglucoside, n-dodecylglucoside, n-dodecylmaltoside, octanoyl-N-methylglucamide, decanoyl-N-methylglucamide, Triton X-100, Triton X-114, Thesit, Isotridecypoly(ethylene glycol ether)n, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

In various embodiments of the above assay methods of the present invention, it may be desirable to immobilize DRD3 (or a DRD3 target molecule) to facilitate separation of complexed from uncomplexed forms of one or both of the proteins, as well as to accommodate automation of the assay. Binding of a test compound to DRD3, or interaction of DRD3 with a target molecule in the presence and absence of a candidate compound, can be accomplished in any vessel suitable for containing the reactants. Examples of such vessels include microtitre plates, test tubes, and micro-centrifuge tubes. In one embodiment, a fusion protein can be provided which adds a domain that allows one or both of the proteins to be bound to a matrix. For example, glutathione-S-transferase (GST) fusion proteins or glutathione-S-transferase fusion proteins can be adsorbed onto glutathione sepharose beads (Sigma Chemical; St. Louis, Mo.) or glutathione derivatized microtitre plates, which are then combined with the test compound or the test compound and either the non-adsorbed target protein or DRD3, and the mixture incubated under conditions conducive to complex formation (e.g., at physiological conditions for salt and pH). Following incubation, the beads or microtitre plate wells are washed to remove any unbound components and complex formation is measured either directly or indirectly, for example, as described above. Alternatively, the complexes can be dissociated from the matrix, and the level of binding or activity of DRD3 can be determined using standard techniques.

Other techniques for immobilizing proteins on matrices can also be used in the screening assays of the invention. For example, either DRD3 or its target molecule can be immobilized utilizing conjugation of biotin and streptavidin. Biotinylated polypeptide of the invention or target molecules can be prepared from biotin-NHS (N-hydroxy-succinimide) using techniques well known in the art (e.g., biotinylation kit, Pierce Chemicals; Rockford, Ill.), and immobilized in the wells of streptavidin-coated plates (Pierce Chemical). Alternatively, antibodies reactive with DRD3 or target molecules but which do not interfere with binding of the polypeptide of the invention to its target molecule can be derivatized to the wells of the plate, and unbound target or polypeptide of the invention trapped in the wells by antibody conjugation. Methods for detecting such complexes, in addition to those described above for the GST-immobilized complexes, include immunodetection of complexes using antibodies reactive with DRD3 or target molecule, as well as enzyme-linked assays which rely on detecting an enzymatic activity associated with DRD3 or target molecule.

The screening assay can also involve monitoring the expression of DRD3. For example, regulators of expression of DRD3 can be identified in a method in which a cell is contacted with a candidate compound and the expression of DRD3 protein or mRNA in the cell is determined. The level of expression of DRD3 protein or mRNA the presence of the candidate compound is compared to the level of expression of DRD3 protein or mRNA in the absence of the candidate compound. The candidate compound can then be identified as a regulator of expression of DRD3 based on this comparison. For example, when expression of DRD3 protein or mRNA protein is greater (statistically significantly greater) in the presence of the candidate compound than in its absence, the candidate compound is identified as a stimulator of DRD3 protein or mRNA expression. Alternatively, when expression of DRD3 protein or mRNA is less (statistically significantly less) in the presence of the candidate compound than in its absence, the candidate compound is identified as an inhibitor of DRD3 protein or mRNA expression. The level of DRD3 protein or mRNA expression in the cells can be determined by methods described below.

*Binding Assays*

For binding assays, the test compound is preferably a small molecule which binds to and occupies the active site of DRD3 polypeptide, thereby making the ligand binding site inaccessible to substrate such that normal biological activity is prevented. Examples of such small molecules include, but are not limited to, small peptides or peptide-like molecules. Potential ligands which bind to a polypeptide of the invention include, but are not limited to, the natural ligands of known DRD3 GPCRs and analogues or derivatives thereof.

In binding assays, either the test compound or the DRD3 polypeptide can comprise a detectable label, such as a fluorescent, radioisotopic, chemiluminescent, or enzymatic label, such as horseradish peroxidase, alkaline phosphatase, or luciferase. Detection of a test compound which is bound to DRD3 polypeptide can then be accomplished, for example, by direct counting of radioemmission, by scintillation counting, or by determining conversion of an appropriate substrate to a detectable product. Alternatively, binding of a test compound to a DRD3 polypeptide can be determined without labeling either of the interactants. For example, a microphysiometer can be used to detect binding of a test compound with a DRD3 polypeptide. A microphysiometer (e.g., Cytosensor™) is an analytical instrument that measures the rate at which a cell acidifies its environment using a light-addressable potentiometric sensor (LAPS). Changes in this acidification rate can be used as an indicator of the interaction between a test compound and DRD3 [Haseloff, (1988)].

Determining the ability of a test compound to bind to DRD3 also can be accomplished using a technology such as real-time Bimolecular Interaction Analysis (BIA) [McConnell, (1992); Sjolander, (1991)]. BIA is a technology for studying biospecific interactions in real time, without labeling any of the interactants *(e.g.,* BIAcore™). Changes in the optical phenomenon surface plasmon resonance (SPR) can be used as an indication of real-time reactions between biological molecules.

In yet another aspect of the invention, a DRD3-like polypeptide can be used as a "bait protein" in a two-hybrid assay or three-hybrid assay [Szabo, (1995); U.S. 5,283,317), to identify other proteins which bind to or interact with DRD3 and modulate its activity.

The two-hybrid system is based on the modular nature of most transcription factors, which consist of separable DNA-binding and activation domains. Briefly, the assay utilizes two different DNA constructs. For example, in one construct, polynucleotide encoding DRD3 can be fused to a polynucleotide encoding the DNA binding domain of a known transcription factor (*e.g.,* GAL-4). In the other construct a DNA sequence that encodes an unidentified protein ("prey" or "sample") can be fused to a polynucleotide that codes for the activation domain of the known transcription factor. If the "bait" and the "prey" proteins are able to interact *in vivo* to form an protein-dependent complex, the DNA-binding and activation domains of the transcription factor are brought into close proximity. This proximity allows transcription of a reporter gene (e.g., LacZ), which is operably linked to a transcriptional regulatory site responsive to the transcription factor. Expression of the reporter gene can be detected, and cell colonies containing the functional transcription factor can be isolated and used to obtain the DNA sequence encoding the protein which interacts with DRD3.

It may be desirable to immobilize either the DRD3 (or polynucleotide) or the test compound to facilitate separation of the bound form from unbound forms of one or both of the interactants, as well as to accommodate automation of the assay. Thus, either the DRD3-like polypeptide (or polynucleotide) or the test compound can be bound to a solid support. Suitable solid supports include, but are not limited to, glass or plastic slides, tissue culture plates, microtiter wells, tubes, silicon chips, or particles such as beads (including, but not limited to, latex, polystyrene, or glass beads). Any method known in the art can be used to attach DRD3-like polypeptide (or polynucleotide) or test compound to a solid support, including use of covalent and noncovalent linkages, passive absorption, or pairs of binding moieties attached respectively to the polypeptide (or polynucleotide) or test compound and the solid support. Test compounds are preferably bound to the solid support in an array, so that the location of individual test compounds can be tracked. Binding of a test compound to DRD3 (or a polynucleotide encoding for DRD3) can be accomplished in any vessel suitable for containing the reactants. Examples of such vessels include microtiter plates; test tubes, and microcentrifuge tubes.

In one embodiment, DRD3 is a fusion protein comprising a domain that allows binding of DRD3 to a solid support. For example, glutathione-S-transferase fusion proteins can be adsorbed onto glutathione sepharose beads (Sigma Chemical; St.

Louis, Mo.) or glutathione derivatized microtiter plates, which are then combined with the test compound or the test compound and the non-adsorbed DRD3; the mixture is then incubated under conditions conducive to complex formation (e.g., at physiological conditions for salt and pH). Following incubation, the beads or microtiter plate wells are washed to remove any unbound components. Binding of the interactants can be determined either directly or indirectly, as described above. Alternatively, the complexes can be dissociated from the solid support before binding is determined.

Other techniques for immobilizing proteins or polynucleotides on a solid support also can be used in the screening assays of the invention. For example, either DRD3 (or a polynucleotide encoding DRD3) or a test compound can be immobilized utilizing conjugation of biotin and streptavidin. Biotinylated DRD3 (or a polynucleotide encoding biotinylated DRD3) or test compounds can be prepared from biotin-NHS (N-hydroxysuccinimide) using techniques well known in the art (e.g., biotinylation kit, Pierce Chemicals, Rockford, I11.) and immobilized in the wells of streptavidin-coated plates (Pierce Chemical). Alternatively, antibodies which specifically bind to DRD3, polynucleotide, or a test compound, but which do not interfere with a desired binding site, such as the active site of DRD3, can be derivatized to the wells of the plate. Unbound target or protein can be trapped in the wells by antibody conjugation.

Methods for detecting such complexes, in addition to those described above for the GST-immobilized complexes, include immunodetection of complexes using antibodies which specifically bind to DRD3 polypeptide or test compound, enzyme-linked assays which rely on detecting an activity of DRD3 polypeptide, and SDS gel electrophoresis under non-reducing conditions.

Screening for test compounds which bind to a DRD3 polypeptide or polynucleotide also can be carried out in an intact cell. Any cell which comprises a DRD3 polypeptide or polynucleotide can be used in a cell-based assay system. A DRD3 polynucleotide can be naturally occurring in the cell or can be introduced using techniques such as those described above. Binding of the test compound to DRD3 or a polynucleotide encoding DRD3 is determined as described above.

*Functional Assays*

Test compounds can be tested for the ability to increase or decrease DRD3 activity of a DRD3 polypeptide. The DRD3 activity can be measured, for example, using methods described in the specific examples, below. DRD3 activity can be measured after contacting either a purified DRD3, a cell membrane preparation, or an intact cell with a test compound. A test compound which decreases DRD3 activity by at least about 10, preferably about 50, more preferably about 75, 90, or 100% is identified as a potential agent for decreasing DRD3 activity. A test compound which increases DRD3 activity by at least about 10, preferably about 50, more preferably about 75, 90, or 100% is identified as a potential agent for increasing DRD3 activity.

One such screening procedure involves the use of melanophores which are transfected to express DRD3. Such a screening technique is described in PCT WO 92/01810 published Feb. 6, 1992. Thus, for example, such an assay may be employed for screening for a compound which inhibits activation of the receptor polypeptide of the present invention by contacting the melanophore cells which encode the receptor with both the receptor ligand and a compound to be screened. Inhibition of the signal generated by the ligand indicates that a compound is a potential antagonist for the receptor, *i.e.,* inhibits activation of the receptor. The screen may be employed for identifying a compound which activates the receptor by contacting such cells with compounds to be screened and determining whether each compound generates a signal, *i.e.,* activates the receptor.

Other screening techniques include the use of cells which express DRD3 (for example, transfected CHO cells) in a system which measures extracellular pH changes caused by receptor activation [Iwabuchi , (1993)]. For example, compounds may be contacted with a cell which expresses the receptor polypeptide of the present invention and a second messenger response, *e.g.,* signal transduction or pH changes, can be measured to determine whether the potential compound activates or inhibits the receptor. Another such screening technique involves introducing RNA encoding DRD3 into *Xenopus* oocytes to transiently express the receptor. The receptor oocytes can then be contacted with the receptor ligand and a compound to be screened, followed by detection of inhibition or activation of a calcium signal in the case of screening for compounds which are thought to inhibit activation of the receptor.

Another screening technique involves expressing DRD3 in cells in which the receptor is linked to a phospholipase C or D. Such cells include endothelial cells, smooth muscle cells, embryonic kidney cells, etc. The screening may be accomplished as described above by quantifying the degree of activation of the receptor from changes in the phospholipase activity.

*Gene Expression*

In another embodiment, test compounds which increase or decrease DRD3 gene expression are identified. As used herein, the term "correlates with expression of a polynucleotide" indicates that the detection of the presence of nucleic acids, the same or related to a nucleic acid sequence encoding DRD3, by northern analysis or relatime PCR is indicative of the presence of nucleic acids encoding DRD3 in a sample, and thereby correlates with expression of the transcript from the polynucleotide encoding DRD3. The term "microarray", as used herein, refers to an array of distinct polynucleotides or oligonucleotides arrayed on a substrate, such as paper, nylon or any other type of membrane, filter, chip, glass slide, or any other suitable solid support. A DRD3 polynucleotide is contacted with a test compound, and the expression of an RNA or polypeptide product of DRD3 polynucleotide is determined. The level of expression of appropriate mRNA or polypeptide in the presence of the test compound is compared to the level of expression of mRNA or polypeptide in the absence of the test compound. The test compound can then be identified as a regulator of expression based on this comparison. For example, when expression of mRNA or polypeptide is greater in the presence of the test compound than in its absence, the test compound is identified as a stimulator or enhancer of the mRNA or polypeptide expression. Alternatively, when expression of the mRNA or polypeptide is less in the presence of the test compound than in its absence, the test compound is identified as an inhibitor of the mRNA or polypeptide expression.

The level of DRD3 mRNA or polypeptide expression in the cells can be determined by methods well known in the art for detecting mRNA or polypeptide. Either qualitative or quantitative methods can be used. The presence of polypeptide products of DRD3 polynucleotide can be determined, for example, using a variety of techniques known in the art, including immunochemical methods such as radioimmunoassay, Western blotting, and immunohistochemistry. Alternatively, polypeptide synthesis can be determined *in vivo,* in a cell culture, or in an *in vitro* translation system by detecting incorporation of labelled amino acids into DRD3.

Such screening can be carried out either in a cell-free assay system or in an intact cell. Any cell which expresses DRD3 polynucleotide can be used in a cell-based assay system. The DRD3 polynucleotide can be naturally occurring in the cell or can be introduced using techniques such as those described above. Either a primary culture or an established cell line can be used.

*Test Compounds*

Suitable test compounds for use in the screening assays of the invention can be obtained from any suitable source, e.g., conventional compound libraries. The test compounds can also be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds [Lam, (1997)]. Examples of methods for the synthesis of molecular libraries can be found in the art. Libraries of compounds may be presented in solution or on beads, bacteria, spores, plasmids or phage.

**Therapeutic Indications and Methods**

It was found by the present applicant that DRD3 is expressed in various human tissues.

*Cardiovascular Disorders*

Heart failure is defined as a pathophysiological state in which an abnormality of cardiac function is responsible for the failure of the heart to pump blood at a rate commensurate with the requirement of the metabolizing tissue. It includes all forms of pumping failures such as high-output and low-output, acute and chronic, right-sided or left-sided, systolic or diastolic, independent of the underlying cause.

Myocardial infarction (MI) is generally caused by an abrupt decrease in coronary blood flow that follows a thrombotic occlusion of a coronary artery previously narrowed by arteriosclerosis. MI prophylaxis (primary and secondary prevention) is included as well as the acute treatment of MI and the prevention of complications.

Ischemic diseases are conditions in which the coronary flow is restricted resulting in a perfusion which is inadequate to meet the myocardial requirement for oxygen. This group of diseases includes stable angina, unstable angina and asymptomatic ischemia.

Peripheral vascular diseases are defined as vascular diseases in which arterial and/or venous flow is reduced resulting in an imbalance between blood supply and tissue oxygen demand. It includes chronic peripheral arterial occlusive disease (PAOD), acute arterial thrombosis and embolism, inflammatory vascular disorders, Raynaud's phenomenon and venous disorders.

Cardiovascular diseases include but are not limited to heart failure, myocardial infarction, ischemic diseases of the heart, and peripheral vascular diseases,

The human DRD3 is highly expressed in the following cardiovascular related tissues: heart atrium. (left), aorta, artery, coronary artery, vein, HUVEC cells. Additionally the activity of the human DRD3 can be modulated to treat the above-mentioned cardiovascular diseases.

*Applications*

The present invention provides for both prophylactic and therapeutic methods for heart failure, ischemic diseases of the heart, myocardial infarction and peripheral vascular diseases.

The regulatory method of the invention involves contacting a cell with an agent that modulates one or more of the activities of DRD3. An agent that modulates activity can be an agent as described herein, such as a nucleic acid or a protein, a naturally-occurring cognate ligand of the polypeptide, a peptide, a peptidomimetic, or any small molecule. In one embodiment, the agent stimulates one or more of the biological activities of DRD3. Examples of such stimulatory agents include the active DRD3 and nucleic acid molecules encoding a portion of DRD3. In another embodiment, the agent inhibits one or more of the biological activities of DRD3. Examples of such inhibitory agents include antisense nucleic acid molecules and antibodies. These regulatory methods can be performed in vitro (e.g., by culturing the cell with the agent) or, alternatively, in vivo (e.g, by administering the agent to a subject). As such, the present invention provides methods of treating an individual afflicted with a disease or disorder characterized by unwanted expression or activity of DRD3 or a protein in the DRD3 signaling pathway. In one embodiment, the method involves administering an agent like any agent identified or being identifiable by a screening assay as described herein, or combination of such agents that modulate say upregulate or downregulate the expression or activity of DRD3 or of any protein in the DRD3 signaling pathway. In another embodiment, the method involves administering a regulator of DRD3 as therapy to compensate for reduced or undesirably low expression or activity of DRD3 or a protein in the DRD3 signaling pathway.

Stimulation of activity or expression of DRD3 is desirable in situations in which activity or expression is abnormally low and in which increased activity is likely to have a beneficial effect. Conversely, inhibition of activity or expression of DRD3 is desirable in situations in which activity or expression of DRD3 is abnormally high and in which decreasing its activity is likely to have a beneficial effect.

This invention is further illustrated by the following examples which should not be construed as limiting. The contents of all references, patents and published patent applications cited throughout this application are hereby incorporated by reference.

## Pharmaceutical Compositions

This invention further pertains to novel agents identified by the above-described screening assays and uses thereof for treatments as described herein.

The nucleic acid molecules, polypeptides, and antibodies (also referred to herein as "active compounds") used in the invention can be incorporated into pharmaceutical compositions suitable for administration. Such compositions typically comprise the nucleic acid molecule, protein, or antibody and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated Supplementary active compounds can also be incorporated into the compositions.

The invention includes use of a DRD3 polynucleotide or polypeptide, or, an antibody, a ribozyme or an antisense oligonucleotide which regulate the activity of a DRD3 polypeptide for the preparation of a pharmaceutical preparation useful in the treatment of heart failure, ischemic diseases of the heart, myocardial infarction or peripheral vascular diseases as well as methods for preparing such compositions by combining one or more such regulators and a pharmaceutically acceptable carrier.

An antagonist of DRD3 may be produced using methods which are generally known in the art. In particular, purified DRD3 may be used to produce antibodies or to screen libraries of pharmaceutical agents to identify those which specifically bind DRD3. Antibodies to DRD3 may also be generated using methods that are well known in the art. Such antibodies may include, but are not limited to, polyclonal, monoclonal, chimeric, single chain antibodies, Fab fragments, and fragments produced by a Fab expression library. Neutralizing antibodies like those which inhibit dimer formation are especially preferred for therapeutic use.

In another embodiment of the invention, the polynucleotides encoding DRD3, or any fragment or complement thereof, may be used for therapeutic purposes. In one aspect, the complement of the polynucleotide encoding DRD3 may be used in situations in which it would be desirable to block the transcription of the mRNA. In particular, cells may be transformed with sequences complementary to polynucleotides encoding DRD3. Thus, complementary molecules or fragments may be used to modulate DRD3 activity, or to achieve regulation of gene function. Such technology is now well known in the art, and sense or antisense oligonucleotides or larger fragments can be designed from various locations along the coding or control regions of sequences encoding DRD3.

Expression vectors derived from retroviruses, adenoviruses, or herpes or vaccinia viruses, or from various bacterial plasmids, may be used for delivery of nucleotide sequences to the targeted organ, tissue, or cell population. Methods which are well known to those skilled in the art can be used to construct vectors which will express nucleic acid sequence complementary to the polynucleotides of the gene encoding DRD3. These techniques are described, for example, in [Scott and Smith (1990) Science 249:386-390].

Any of the therapeutic methods described above may be applied to any subject in need of such therapy, including, for example, mammals such as dogs, cats, cows, horses, rabbits, monkeys, and most preferably, humans.

Disclosed is the administration of a pharmaceutical composition containing DRD3 in conjunction with a pharmaceutically acceptable carrier, for any of the therapeutic effects discussed above. Such pharmaceutical compositions may consist of DRD3, antibodies to DRD3, and mimetics, agonists, antagonists, or inhibitors of DRD3. The compositions may be administered alone or in combination with at least one other agent, such as a stabilizing compound, which may be administered in any sterile, biocompatible pharmaceutical carrier including, but not limited to, saline, buffered saline, dextrose, and water. The compositions may be administered to a patient alone, or in combination with other agents, drugs or hormones.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersions. For intravenous administration, suitable carriers include physiological saline, bacteriostatic water, Cremophor EM™ (BASF, Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, a pharmaceutically acceptable polyol like glycerol, propylene glycol, liquid polyetheylene glycol, and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin. Sterile injectable solutions can be prepared by incorporating the active compound (e.g., a polypeptide or antibody) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed.

Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressurized container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Another technique for drug screening which may be used provides for high throughput screening of compounds having suitable binding affinity to the protein of interest as described in published PCT application WO84/03564. In this method, large numbers of different small test compounds are synthesized on a solid substrate, such as plastic pins or some other surface. The test compounds are reacted with DRD3, or fragments thereof, and washed. Bound DRD3 is then detected, by methods well known in the art. Purified DRD3 can also be coated directly onto plates for use in the aforementioned drug screening techniques. Alternatively, non-neutralizing antibodies can be used to capture the peptide and immobilize it on a solid support.

In another embodiment, one may use competitive drug screening assays in which neutralizing antibodies capable of binding DRD3 specifically compete with a test-compound for binding DRD3. In this manner, antibodies can be used to detect the presence of any peptide which shares one or more antigenic determinants with DRD3.

**Determination of a Therapeutically Effective Dose**

The determination of a therapeutically effective dose is well within the capability of those skilled in the art. A therapeutically effective dose refers to that amount of active ingredient which increases or decreases DRD3 activity relative to DRD3 activity which occurs in the absence of the therapeutically effective dose. For any compound, the therapeutically effective dose can be estimated initially either in cell culture assays or in animal models, usually mice, rabbits, dogs, or pigs. The animal model also can be used to determine the appropriate concentration range and route of administration. Such information can then be used to determine useful doses and routes for administration in humans.

Therapeutic efficacy and toxicity, e.g., $ED_{50}$ (the dose therapeutically effective in 50% of the population) and $LD_{50}$ (the dose lethal to 50% of the population), can be determined by standard pharmaceutical procedures in cell cultures or experimental animals. The dose ratio of toxic to therapeutic effects is the therapeutic index, and it can be expressed as the ratio, $LD_{50}/ED_{50}$. Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the $ED_{50}$ with little or no toxicity. The dosage varies within this range depending upon the dosage form employed, sensitivity of the patient, and the route of administration. The exact dosage will be determined by the practitioner, in light of factors related to the subject that requires treatment. Dosage and administration are adjusted to provide sufficient levels of the active ingredient or to maintain the desired effect. Factors which can be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions can be administered every 3 to 4 days, every week, or once every two weeks depending on the half-life and clearance rate of the particular formulation.

Normal dosage amounts can vary from 0.1 micrograms to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc. If the reagent is a single-chain antibody, polynucleotides encoding the antibody can be constructed and introduced into a cell either *ex vivo* or *in vivo* using well-established techniques including, but not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, "gene gun", and DEAE- or calcium phosphate-mediated transfection.

If the expression product is mRNA, the reagent is preferably an antisense oligonucleotide or a ribozyme. Polynucleotides which express antisense oligonucleotides or ribozymes can be introduced into cells by a variety of methods, as described above. Preferably, a reagent reduces expression of DRD3 gene or the activity of DRD3 by at least about 10, preferably about 50, more preferably about 75, 90, or 100% relative to the absence of the reagent. The effectiveness of the mechanism chosen to decrease the level of expression of DRD3 gene or the activity of DRD3 can be assessed using methods well known in the art, such as hybridization of nucleotide probes to DRD3-specific mRNA, quantitative RT-PCR, immunologic detection of DRD3, or measurement of DRD3 activity.

In any of the embodiments described above, any of the pharmaceutical compositions of the invention can be administered in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects. Any of the therapeutic methods described above can be applied to any subject in need of such therapy, including, for example, mammals such as dogs, cats, cows, horses, rabbits, monkeys, and most preferably, humans.

Nucleic acid molecules used in the invention are those nucleic acid molecules which are contained in a group of nucleic acid molecules consisting of (i) nucleic acid molecules encoding a polypeptide comprising the amino acid sequence of SEQ ID NO: 2, (ii) nucleic acid molecules comprising the sequence of SEQ ID NO: 1, (iii) nucleic acid molecules having the sequence of SEQ ID NO: 1, (iv)nucleic acid molecules the complementary strand of which hybridizes under stringent conditions to a nucleic acid molecule of (i), (ii), or (iii); and (v) nucleic acid molecules the sequence of which differs from the sequence of a nucleic acid molecule of (iii) due to the degeneracy of the genetic code, wherein the polypeptide encoded by said nucleic acid molecule has DRD3 activity.

Polypeptides used in the invention are those polypeptides which are contained in a group of polypeptides consisting of (i) polypeptides having the sequence of SEQ ID NO: 2, (ii) polypeptides comprising the sequence of SEQ ID NO: 2, (iii) polypeptides encoded by nucleic acid molecules of the invention and (iv) polypeptides which show at least 99%, 98%, 95%, 90%, or 80% homology with a polypeptide of (i), (ii), or (iii), wherein said purified polypeptide has DRD3 activity.

An object of the invention is a method of screening for therapeutic agents useful in the treatment of a disease comprised in a group of diseases consisting of heart failure, ischemic diseases of the heart, myocardial infarction and peripheral vascular diseases in a mammal comprising the steps of (i) contacting a test compound with a DRD3 polypeptide, (ii) detect binding of said test compound to said DRD3 polypeptide. E.g., compounds that bind to the DRD3 polypeptide are identified potential therapeutic agents for such a disease.

Another object of the invention is a method of screening for therapeutic agent useful in the treatment of a disease comprised in a group of diseases consisting of heart failure, ischemic diseases of the heart, myocardial infarction and peripheral vascular diseases in a mammal comprising the steps of (i) determining the activity of a DRD3 polypeptide at a certain concentration of a test compound or in the absence of said test compound, (ii) determining the activity of said polypeptide at a different concentration of said test compound. E.g., compounds that lead to a difference in the activity of the DRD3 polypeptide in (i) and (ii) are identified potential therapeutic agents for such a disease.

Another object of the invention is a method of screening for therapeutic agents useful in the treatment of a disease comprised in a group of diseases consisting of heart failure, ischemic diseases of the heart, myocardial infarction and peripheral vascular diseases in a mammal comprising the steps of (i) determining the activity of a DRD3 polypeptide at a certain concentration of a test compound, (ii) determining the activity of a DRD3 polypeptide at the presence of a compound known to be a regulator of a DRD3 polypeptide. E.g., compounds that show similar effects on the activity of the DRD3 polypeptide in (i) as compared to compounds used in (ii) are identified potential therapeutic agents for such a disease.

Other objects of the invention are methods of the above, wherein the step of contacting is in or at the surface of a cell.

Other objects of the invention are methods of the above, wherein the cell is in vitro.

Other objects of the invention are methods of the above, wherein the step of contacting is in a cell-free system.

Other objects of the invention are methods of the above, wherein the polypeptide is coupled to a detectable label.

Other objects of the invention are methods of the above, wherein the compound is coupled to a detectable label.

Other objects of the invention are methods of the above, wherein the test compound displaces a ligand which is first bound to the polypeptide.

Other objects of the invention are methods of the above, wherein the polypeptide is attached to a solid support.

Other objects of the invention are methods of the above, wherein the compound is attached to a solid support.

Another object of the invention is a method of screening for therapeutic agents useful in the treatment of a disease comprised in a group of diseases consisting of heart failure, ischemic diseases of the heart, myocardial infarction and peripheral vascular diseases in a mammal comprising the steps of (i) contacting a test compound with a DRD3 polynucleotide, (ii) detect binding of said test compound to said DRD3 polynucleotide. Compounds that, e.g., bind to the DRD3 polynucleotide are potential therapeutic agents for the treatment of such diseases.

Another object of the invention is the method of the above, wherein the nucleic acid molecule is RNA.

Another object of the invention is a method of the above, wherein the contacting step is in or at the surface of a cell.

Another object of the invention is a method of the above, wherein the contacting step is in a cell-free system.

Another object of the invention is a method of the above, wherein the polynucleotide is coupled to a detectable label.

Another object of the invention is a method of the above, wherein the test compound is coupled to a detectable label. amount of DRD3 polynucleotide in said test mammal as compared to a diseased mammal.

Another object of the invention is a pharmaceutical composition for the treatment of a disease comprised in a group of diseases consisting of heart failure, ischemic diseases of the heart, myocardial infarction and peripheral vascular diseases comprising a therapeutic agent which regulates the activity of a DRD3 polypeptide, wherein said therapeutic agent is (i) antisense oligonucleotide, (ii) an antibody, or (iii) a ribozyme.

Another object of the invention is a pharmaceutical composition for the treatment of a disease comprised in a group of diseases consisting of heart failure, ischemic diseases of the heart, myocardial infarction and peripheral vascular diseases in a mammal comprising a DRD3 polynucleotide.

Another object of the invention is a pharmaceutical composition for the treatment of a disease comprised in a group of diseases consisting of heart failure, ischemic diseases of the heart, myocardial infarction and peripheral vascular diseases in a mammal comprising a DRD3 polypeptide.

The examples below are provided to illustrate the subject invention. These examples are provided by way of illustration and are not included for the purpose of limiting the invention.

## Examples

**Example 1:** Search for homologous sequences in public sequence data bases

The degree of homology can readily be calculated by known methods. Preferred methods to determine homology are designed to give the largest match between the sequences tested. Methods to determine homology are codified in publicly available computer programs such as BestFit, BLASTP, BLASTN, and FASTA. The BLAST programs are publicly available from NCBI and other sources in the internet.

For DRD3 the following hits to known sequences were identified by using the BLAST algorithm [Altschul SF, Madden TL, Schaffer AA, Zhang J, Zhang Z, Miller W, Lipman DJ; Nucleic Acids Res 1997 Sep 1; 25(17): 3389-402] and the following set of parameters: matrix = BLOSUM62 and low complexity filter. The following databases were searched: NCBI (non-redundant database) and DERWENT patent database (Geneseq).

The following hits were found:

>emb|CAA01483.1| dopaminergic receptor D3 [Homo sapiens] Length = 400 Score = 807 bits (2084), Expect = 0.0 Identities = 400/400 (100%), Positives = 400/400 (100%)

>ref|NP_000787.1| dopamine receptor D3, isoform a [Homo sapiens] gb|AAA73929.1| d3 dopamine receptor pir||G01977 d3 dopamine receptor - human sp|P35462|D3DR_HUMAN D(3) dopamine receptor Length = 400 Score = 807 bits (2084), Expect = 0.0 Identities = 400/400 (100%), Positives = 400/400 (100%)

>prf||1705199A dopamine D3 receptor Length = 400 Score = 805 bits (2078), Expect = 0.0 Identities = 399/400 (99%), Positives = 399/400 (99%)

>gb|AAB08750.1| dopamine D3 receptor Length = 399 Score = 796 bits (2055), Expect = 0.0 Identities = 396/400 (99%), Positives = 398/400 (99%), Gaps = 1/400 (0%)

>gb|AAA75379.1| D3 dopamine receptor pir||G00013 D3 dopamine receptor - green monkey sp|P52703|D3DR_CERAE D(3) DOPAMINE RECEPTOR Length = 400 Score = 795 bits (2054), Expect = 0.0 Identities = 393/400 (98%), Positives = 395/400 (98%)

>gb|AAL82735.1| dopamine receptor D3 [Cebus apella] Length = 400 Score = 784 bits (2025), Expect = 0.0 Identities = 388/400 (97%), Positives = 392/400 (98%)

>sp|P30728|D3DR_MOUSE D(3) dopamine receptor ref|NP_031903.1| dopamine receptor 3; D3 receptor [Mus musculus] gb|AAB35066.2| dopamine D3 receptor [Mus sp.] pir||I48322 dopamine receptor D3 - mouse prf||2105315A D3 dopamine receptor emb|CAA47691.1| D3 dopamine receptor [Mus musculus] Length = 446 Score = 713 bits (1841), Expect = 0.0 Identities = 363/447 (81%), Positives = 380/447 (84%), Gaps = 48/447 (10%)

>emb|CAA01350.1| dopaminergic receptor D3 [Rattus norvegicus] gb|AAA70477.1| Sequence 3 from Patent US 5407823 Length = 446 Score = 700 bits (1806), Expect = 0.0 Identities = 356/447 (79%), Positives = 375/447 (83%), Gaps = 48/447 (10%)

>ref|NP_058836.1| dopamine receptor D3 [Rattus norvegicus] pir||DYRTD3 dopamine receptor D3 - rat emb|CAA37887.1| dopamine D3 receptor [Rattus norvegicus] prf||1614344A dopamine receptor D3 sp|P19020|D3DR_RAT D(3) dopamine receptor Length = 446 Score = 700 bits (1806), Expect = 0.0 Identities = 356/447 (79%), Positives = 375/447 (83%), Gaps = 48/447 (10%)

>emb|CAA01349.1| domaminergic receptor D3 (variant) [Rattus norvegicus] gb|AAA70476.1| Sequence 2 from Patent US 5407823 Length = 446 Score = 699 bits (1803), Expect = 0.0 Identities = 355/447 (79%), Positives = 375/447 (83%), Gaps = 48/447 (10%)

>gb|AAA41076.1| dopamine receptor D3 Length = 454 Score = 691 bits (1784), Expect = 0.0 Identities = 355/455 (78%), Positives = 375/455 (82%), Gaps = 56/455 (12%)

>gb|AAB45999.1| Sequence 20 from patent US 5594108 gb|AAA71265.1| Sequence 20 from patent US 5422265 Length = 444 Score = 690 bits (1781), Expect = 0.0 Identities = 353/447 (78%), Positives = 373/447 (82%), Gaps = 50/447 (11 %)

>pir||A48258 dopamine receptor variant D3nf- (schizophrenia) - human gb|AAA03543.1| dopamine D3 receptor ref|NP_387512.1| dopamine receptor D3, isoform e [Homo sapiens] Length = 342 Score = 580 bits (1495), Expect = e-164 Identities = 288/289 (99%), Positives = 288/289 (99%)

>gb|AAB11090.1| Sequence 27 from patent US 5508384 Length = 317 Score = 577 bits (1487), Expect = e-163 Identities = 309/372 (83%), Positives = 314/372 (84%), Gaps = 56/372 (15%)

>ref|NP_387508.1| dopamine receptor D3, isoform c [Homo sapiens] Length = 327 Score = 476 bits (1225), Expect = e-133 Identities = 237/237 (100%), Positives = 237/237 (100%)

>gb|AAN87173.1| dopamine D3 receptor [Danio rerio] Length = 454 Score = 440 bits (1132), Expect = e-122 Identities = 258/449 (57%), Positives = 301/449 (66%), Gaps = 65/449 (14%)

>gb|AAG34497.1| dopamine D2 receptor short isoform [Canis familiaris] Length = 414 Score = 395 bits (1014), Expect = e-109 Identities = 221/399 (55%), Positives = 270/399 (67%), Gaps = 24/399 (6%)

>gb|AAG34495.1| dopamine D2 receptor short isoform [Canis familiaris] Length = 414 Score = 394 bits (1012), Expect = e-108 Identities = 221/399 (55%), Positives = 269/399 (67%), Gaps = 24/399 (6%)

>emb|CAC87873.1| dopamine D2 receptor 1 [Oncorhynchus mykiss] Length = 461 Score = 394 bits (1011), Expect = e-108 Identities = 228/447 (51%), Positives = 288/447 (64%), Gaps = 66/447 (14%)

>prf||1502338A D2 dopamine receptor gb|AAA41075.1| dopamine receptor subtype D2 Length = 415 Score = 393 bits (1009), Expect = e-108 Identities = 224/402 (55%), Positives = 273/402 (67%), Gaps = 29/402 (7%)

>emb|CAA35970.1| dopamine receptor D2 [Bos taurus] pir||DYBOD2 dopamine receptor D2 - bovine ref|NP_776468.1| dopamine receptor D2 [Bos taurus] sp|P20288|D2DR_BOVIN D(2) dopamine receptor Length = 444 Score = 391 bits (1004), Expect = e-107 Identities = 229/441 (51%), Positives = 278/441 (62%), Gaps = 65/441 (14%)

>gb|AAB20571.1| D2 dopamine receptor [Homo sapiens] ref|NP_057658.2| dopamine receptor D2 isoform short [Homo sapiens] Length = 414 Score = 391 bits (1004), Expect = e-107 Identities = 221/399 (55%), Positives = 268/399 (66%), Gaps = 24/399 (6%)

**Example 2:** Expression profiling

Total cellular RNA was isolated from cells by one of two standard methods: 1) guanidine isothiocyanate/Cesium chloride density gradient centrifugation [Kellogg, (1990)] ; or with the Tri-Reagent protocol according to the manufacturer's specifications (Molecular Research Center, Inc., Cincinatti, Ohio). Total RNA prepared by the Tri-reagent protocol was treated with DNAse I to remove genomic DNA contamination.

For relative quantitation of the mRNA distribution of DRD3, total RNA from each cell or tissue source was first reverse transcribed. 85 μg of total RNA was reverse transcribed using 1 μmole random hexamer primers, 0.5 mM each of dATP, dCTP, dGTP and dTTP (Qiagen, Hilden, Germany), 3000 U RnaseQut (Invitrogen, Groningen, Netherlands) in a final volume of 680 μl. The first strand synthesis buffer and Omniscript reverse transcriptase (2 u/μl) were from (Qiagen, Hilden, Germany). The reaction was incubated at 37°C for 90 minutes and cooled on ice. The volume was adjusted to 6800 μl with water, yielding a final concentration of 12.5 ng/μl of starting RNA.

For relative quantitation of the distribution of DRD3 mRNA in cells and tissues the Applied Biosystems 7900 HT Sequence Detection system or BioradiCycler was used according to the manufacturer's specifications and protocols. PCR reactions were set up to quantitate DRD3 and the housekeeping genes HPRT (hypoxanthine phosphoribosyltransferase), GAPDH (glyceraldehyde-3-phosphate dehydrogenase), β-actin, and others. Forward and reverse primers and probes for DRD3 were designed using the Perkin Elmer ABI Primer Express™ software and were synthesized by TibMolBiol (Berlin, Germany). The DRD3 forward primer sequence was: Primer1 (SEQ ID NO: 3). The DRD3 reverse primer sequence was Primer2 (SEQ ID NO: 4). Probe1 (SEQ ID NO: 5), labelled with FAM (carboxyfluorescein succinimidyl ester) as the reporter dye and TAMRA (carboxytetramethylrhodamine) as the quencher, is used as a probe for DRD3. The following reagents were prepared in a total of 25 μl : 1x TaqMan buffer A, 5.5 mM $MgCl_2$, 200 nM of dATP, dCTP, dGTP, and dUTP, 0.025 U/μl AmpliTaq Gold™, 0.01 U/ μl AmpErase and Probe1 (SEQ ID NO: 4), DRD3 forward and reverse primers each at 200 nM, 200 nM DRD3 FAM/TAMRA-labelled probe, and 5 μl of template cDNA. Thermal cycling parameters were 2 min at 50°C, followed by 10 min at 95°C, followed by 40 cycles of melting at 95°C for 15 sec and annealing/extending at 60°C for 1 min.

*Calculation of corrected CT values*

The CT (threshold cycle) value is calculated as described in the "Quantitative determination of nucleic acids" section. The CF-value (factor for threshold cycle correction) is calculated as follows:

1. PCR reactions were set up to quantitate the housekeeping genes (HKG) for each cDNA sample.

2. $CT_{HKG}$-values (threshold cycle for housekeeping gene) were calculated as described in the "Quantitative determination of nucleic acids" section.

3. $CT_{HKG}$-mean values (CT mean value of all HKG tested on one cDNAs) of all HKG for each cDNA are calculated (n = number of HKG):

$$CT_{HKG\text{-}n}\text{-mean value} = (CT_{HKG1}\text{-value} + CT_{HKG2}\text{-value} + \ldots + CT_{HKG\text{-}n}\text{-value}) / n$$

4.

$$CT_{pannel}\text{ mean value (CT mean value of all HKG in all tested cDNAs)} =$$

$$(CT_{HKG1}\text{-mean value} + CT_{HKG2}\text{-mean value} + \ldots + CT_{HKG\text{-}y}\text{-mean value}) / y$$

$$(y = \text{number of cDNAs})$$

5.

$$CF_{cDNA\text{-}n}\text{ (correction factor for cDNA n)} = CT_{pannel}\text{-mean value} - CT_{HKG\text{-}n}\text{-mean value}$$

6.

$$CT_{cDNA\text{-}n}\text{ (CT value of the tested gene for the cDNA n)} + CF_{cDNA\text{-}n}\text{ (correction factor for cDNA n)} = CT_{cor\text{-}cDNA\text{-}n}\text{ (corrected CT value for a gene on cDNA n)}$$

*Calculation of relative expression*

Definition : highest $CT_{cor\text{-}cDNA\text{-}n} \neq 40$ is defined as $CT_{cor\text{-}cDNA}$ [high] Relative Expression = $2^{(CTcor\text{-}cDNA[high]\ -\ CTcor\text{-}cDNA\text{-}n)}$

*Tissues*

The expression of DRD3 was investigated in the tissues listed in table 1.

*Expression profile*

The results of the the mRNA-quantification (expression profiling) is shown in Table 1.

***Table 1:*** *Relative expression of DRD3 in various human tissues.*

| Tissue | Relative Expression |
|---|---|
| fetal heart | 17 |
| heart | 14 |
| pericardium | 102 |
| heart atrium (right) | 25 |
| heart atrium (left) | 292 |
| heart ventricle (left) | 59 |
| interventricular septum | 95 |
| fetal aorta | 10 |
| aorta | 437 |
| artery | 996 |
| coronary artery | 2957 |
| vein | 653 |
| HUVEC cells | 365 |
| | |
| skin | 12 |
| | |
| adrenal gland | 13 |
| thyroid | 0 |
| thyroid tumor | 22 |
| pancreas | 0 |
| pancreas liver cirrhosis | 83 |
| esophagus | 15 |
| esophagus tumor | 154 |
| stomach | 6 |
| stomach tumor | 195 |
| colon | 1 |
| colon tumor | 276 |
| small intestine | 81 |
| ileum | 434 |
| ileum tumor | 205 |
| ileum chronic inflammation | 771 |
| rectum | 3061 |
| salivary gland | 4 |
| fetal liver | 10 |
| liver | 12 |
| liver liver cirrhosis | 1458 |
| liver tumor | 231 |
| HEP G2 cells | 407 |
| | |
| leukocytes (peripheral blood) | 72 |
| Jurkat (T-cells) | 149 |
| bone marrow | 8 |
| erythrocytes | 498 |
| lymphnode | 776 |
| thymus | 10 |
| thrombocytes | 523 |

(continued)

| Tissue | Relative Expression |
|---|---|
| bone marrow stromal cells | 63 |
| bone marrow CD71+ cells | 340 |
| bone marrow CD33+ cells | 3019 |
| bone marrow CD34+ cells | 292 |
| bone marrow CD15+ cells | 495 |
| cord blood CD71+ cells | 195 |
| spleen | 97 |
| spleen liver cirrhosis | 0 |
| | |
| skeletal muscle | 83 |
| adipose | 1 |
| | |
| fetal brain | 72 |
| brain | 74 |
| Alzheimer brain | 1075 |
| cerebellum | 4 |
| cerebellum (right) | 405 |
| cerebellum (left) | 855 |
| cerebral cortex | 471 |
| Alzheimer cerebral cortex | 57 |
| frontal lobe | 1038 |
| Alzheimer brain frontal lobe | 1489 |
| occipital lobe | 1333 |
| parietal lobe | 165 |
| temporal lobe | 82 |
| precentral gyrus | 256 |
| postcentral gyrus | 115 |
| tonsilla cerebelli | 247 |
| vermis cerebelli | 315 |
| pons | 4182 |
| substantia nigra | 729 |
| cerebral meninges | 1924 |
| cerebral peduncles | 198 |
| corpus callosum | 588 |
| hippocampus | 300 |
| thalamus | 1783 |
| dorsal root ganglia | 1641 |
| spinal cord | 65 |
| neuroblastoma SK-N-MC cells | 309 |
| neuroblastoma SH-SY5Y cells | 94 |
| neuroblastoma IMR32 cells | 588 |
| glial tumor H4 cells | 298 |
| glial tumor H4 cells + APP | 218 |
| HEK CNS | 580 |
| HEK CNS + APP | 798 |
| retina | 942 |
| | |
| fetal lung | 24 |
| fetal lung fibroblast IMR-90 cells | 11 |
| lung | 6 |

(continued)

| Tissue | Relative Expression |
|---|---|
| lung right upper lobe | 35 |
| lung right mid lobe | 42 |
| lung right lower lobe | 685 |
| lung tumor | 2628 |
| lung COPD | 322 |
| trachea | 14 |
| | |
| cervix | 0 |
| testis | 214 |
| HeLa cells (cervix tumor) | 2 |
| placenta | 6 |
| uterus | 399 |
| uterus tumor | 103 |
| ovary | 2 |
| ovary tumor | 1859 |
| breast | 164 |
| breast tumor | 505 |
| MDA MB 231 cells (breast tumor) | 123 |
| mammary gland | 4 |
| | |
| prostate | 42 |
| prostate BPH | 51 |
| bladder | 5 |
| ureter | 428 |
| penis | 765 |
| corpus cavernosum | 302 |
| fetal kidney | 19 |
| kidney | 34 |
| kidney tumor | 229 |
| HEK 293 cells | 276 |

**Example 3:** Antisense Analysis

Knowledge of the correct, complete cDNA sequence coding for DRD3 enables its use as a tool for antisense technology in the investigation of gene function. Oligonucleotides, cDNA or genomic fragments comprising the antisense strand of a polynucleotide coding for DRD3 are used either in vitro or in vivo to inhibit translation of the mRNA. Such technology is now well known in the art, and antisense molecules can be designed at various locations along the nucleotide sequences. By treatment of cells or whole test animals with such antisense sequences, the gene of interest is effectively turned off. Frequently, the function of the gene is ascertained by observing behavior at the intracellular, cellular, tissue or organismal level (e.g., lethality, loss of differentiated function, changes in morphology, etc.).

In addition to using sequences constructed to interrupt transcription of a particular open reading frame, modifications of gene expression is obtained by designing antisense sequences to intron regions, promoter/enhancer elements, or even to transacting regulatory genes.

**Example 4:** Expression of DRD3

Expression of DRD3 is accomplished by subcloning the cDNAs into appropriate expression vectors and transfecting the vectors into expression hosts such as, e.g., *E. coli.* In a particular case, the vector is engineered such that it contains a promoter for β-galactosidase, upstream of the cloning site, followed by sequence containing the amino-terminal Methionine and the subsequent seven residues of β-galactosidase. Immediately following these eight residues is an engineered bacteriophage promoter useful for artificial priming and transcription and for providing a number of unique endonuclease restriction sites for cloning.

Induction of the isolated, transfected bacterial strain with Isopropyl-β-D-thiogalactopyranoside (IPTG) using standard methods produces a fusion protein corresponding to the first seven residues of β-galactosidase, about 15 residues of "linker", and the peptide encoded within the cDNA. Since cDNA clone inserts are generated by an essentially random process, there is probability of 33% that the included cDNA will lie in the correct reading frame for proper translation. If the cDNA is not in the proper reading frame, it is obtained by deletion or insertion of the appropriate number of bases using well known methods including in vitro mutagenesis, digestion with exonuclease III or mung bean nuclease, or the inclusion of an oligonucleotide linker of appropriate length.

The DRD3 cDNA is shuttled into other vectors known to be useful for expression of proteins in specific hosts. Oligonucleotide primers containing cloning sites as well as a segment of DNA (about 25 bases) sufficient to hybridize to stretches at both ends of the target cDNA is synthesized chemically by standard methods. These primers are then used to amplify the desired gene segment by PCR. The resulting gene segment is digested with appropriate restriction enzymes under standard conditions and isolated by gel electrophoresis. Alternately, similar gene segments are produced by digestion of the cDNA with appropriate restriction enzymes. Using appropriate primers, segments of coding sequence from more than one gene are ligated together and cloned in appropriate vectors. It is possible to optimize expression by construction of such chimeric sequences.

Suitable expression hosts for such chimeric molecules include, but are not limited to, mammalian cells such as Chinese Hamster Ovary (CHO) and human 293 cells., insect cells such as Sf9 cells, yeast cells such as *Saccharomyces cerevisiae* and bacterial cells such as *E. coli.* For each of these cell systems, a useful expression vector also includes an origin of replication to allow propagation in bacteria, and a selectable marker such as the β-lactamase antibiotic resistance gene to allow plasmid selection in bacteria. In addition, the vector may include a second selectable marker such as the neomycin phosphotransferase gene to allow selection in transfected eukaryotic host cells. Vectors for use in eukaryotic expression hosts require RNA processing elements such as 3' polyadenylation sequences if such are not part of the cDNA of interest.

Additionally, the vector contains promoters or enhancers which increase gene expression. Such promoters are host specific and include MMTV, SV40, and metallothionine promoters for CHO cells; trp, lac, tac and T7 promoters for bacterial hosts; and alpha factor, alcohol oxidase and PGH promoters for yeast. Transcription enhancers, such as the rous sarcoma virus enhancer, are used in mammalian host cells. Once homogeneous cultures of recombinant cells are obtained through standard culture methods, large quantities of recombinantly produced DRD3 are recovered from the conditioned medium and analyzed using chromatographic methods known in the art. For example, DRD3 can be cloned into the expression vector pcDNA3, as exemplified herein. This product can be used to transform, for example, HEK293 or COS by methodology standard in the art. Specifically, for example, using Lipofectamine (Gibco BRL catolog no. 18324-020) mediated gene transfer.

**Example 5:** Isolation of Recombinant DRD3

DRD3 is expressed as a chimeric protein with one or more additional polypeptide domains added to facilitate protein purification. Such purification facilitating domains include, but are not limited to, metal chelating peptides such as histidinetryptophan modules that allow purification on immobilized metals [Appa Rao, 1997] and the domain utilized in the FLAGS extension/affinity purification system (Immunex Corp., Seattle, Washington). The inclusion of a cleavable linker sequence such as Factor Xa or enterokinase (Invitrogen, Groningen, The Netherlands) between the purification domain and the DRD3 sequence is useful to facilitate expression of DRD3.

**Example 6:** Testing of Chimeric GPCRs

Functional chimeric GPCRs are constructed by combining the extracellular receptive sequences of a new isoform with the transmembrane and intracellular segments of a known isoform for test purposes. This concept was demonstrated by Kobilka et al. (1988), Science 240:1310-1316) who created a series of chimeric α2-β2 adrenergic receptors (AR) by inserting progressively greater amounts of α2-AR transmembrane sequence into β2-AR. The binding activity of known agonists changed as the molecule shifted from having more α2 than β2 conformation, and intermediate constructs demonstrated mixed specificity. The specificity for binding antagonists, however, correlated with the source of the domain VII. The importance of T7G domain VII for ligand recognition was also found in chimeras utilizing two yeast α-factor receptors and is significant because the yeast receptors are classified as miscellaneous receptors. Thus, functional role of specific domains appears to be preserved throughout the GPCR family regardless of category.

In parallel fashion, internal segments or cytoplasmic domains from a particular isoform are exchanged with the analogous domains of a known GPCRs and used to identify the structural determinants responsible for coupling the receptors to trimeric G-proteins. A chimeric receptor in which domains V, VI, and the intracellular connecting loop from β2-AR were substituted into α2-AR was shown to bind ligands with α2-AR specificity, but to stimulate adenylate cyclase in the manner of β2-AR. This demonstrates that for adrenergic-type receptors, G-protein recognition is present in domains V and VI and their connecting loop. The opposite situation was predicted and observed for a chimera in which the V->VI loop from α1-AR replaced the corresponding domain on β2-AR and the resulting receptor bound ligands with β2-AR specificity and activated G-protein-mediated phosphatidylinositol turnover in the α1-AR manner. Finally, chimeras constructed from muscarinic receptors also demonstrated that V->VI loop is the major determinant for specificity of G-protein activity.

Chimeric or modified GPCRs containing substitutions in the extracellular and transmembrane regions have shown that these portions of the receptor determine ligand binding specificity. For example, two Serine residues conserved in domain V of all adrenergic and D catecholainine GPCRs are necessary for potent agonist activity. These serines are believed to form hydrogen bonds with the catechol moiety of the agonists within the GPCR binding site. Similarly, an Asp residue present in domain III of all GPCRs which bind biogenic amines is believed to form an ion pair with the ligand amine group in the GPCR binding site.

Functional, cloned GPCRs are expressed in heterologous expression systems and their biological activity assessed. One heterologous system introduces genes for a mammalian GPCR and a mammalian G-protein into yeast cells. The GPCR is shown to have appropriate ligand specificity and affinity and trigger appropriate biological activation (growth arrest and morphological changes) of the yeast cells.

An alternate procedure for testing chimeric receptors is based on the procedure utilizing the purinergic receptor ($P_2$u). Function is easily tested in cultured K562 human leukemia cells because these cells lack $P_2$u receptors. K562 cells are transfected with expression vectors containing either normal or chimeric $P_2$u and loaded with fura-a, fluorescent probe for $Ca^{++}$. Activation of properly assembled and functional $P_2$u receptors with extracellular UTP or ATP mobilizes intracellular $Ca^{++}$ which reacts with fura-a and is measured spectrofluorometrically.

As with the GPCRs above, chimeric genes are created by combining sequences for extracellular receptive segments of any new GPCR polypeptide with the nucleotides for the transmembrane and intracellular segments of the known $P_2$u molecule. Bathing the transfected K562 cells in microwells containing appropriate ligands triggers binding and fluorescent activity defining effectors of the GPCR molecule. Once ligand and function are established, the $P_2$u system is useful for defining antagonists or inhibitors which block binding and prevent such fluorescent reactions.

**Example 7:** Production of DRD3 Specific Antibodies

Two approaches are utilized to raise antibodies to DRD3, and each approach is useful for generating either polyclonal or monoclonal antibodies. In one approach, denatured protein from reverse phase HPLC separation is obtained in quantities up to 75 mg. This denatured protein is used to immunize mice or rabbits using standard protocols; about 100 μg are adequate for immunization of a mouse, while up to 1 mg might be used to immunize a rabbit. For identifying mouse hybridomas, the denatured protein is radioiodinated and used to screen potential murine B-cell hybridomas for those which produce antibody. This procedure requires only small quantities of protein, such that 20 mg is sufficient for labeling and screening of several thousand clones.

In the second approach, the amino acid sequence of an appropriate DRD3 domain, as deduced from translation of the cDNA, is analyzed to determine regions of high antigenicity. Oligopeptides comprising appropriate hydrophilic regions are synthesized and used in suitable immunization protocols to raise antibodies. The optimal amino acid sequences for immunization are usually at the C-terminus, the N-terminus and those intervening, hydrophilic regions of the polypeptide which are likely to be exposed to the external environment when the protein is in its natural conformation.

Typically, selected peptides, about 15 residues in length, are synthesized using an Applied Biosystems Peptide Synthesizer Model 431A using fmoc-chemistry and coupled to keyhole limpet hemocyanin (KLH; Sigma, St. Louis, MO) by reaction with M-maleimidobenzoyl-N-hydroxysuccinimide ester, MBS. If necessary, a cysteine is introduced at the N-terminus of the peptide to permit coupling to KLH. Rabbits are immunized with the peptide-KLH complex in complete Freund's adjuvant. The resulting antisera are tested for antipeptide activity by binding the peptide to plastic, blocking with 1% bovine serum albumin, reacting with antisera, washing and reacting with labeled (radioactive or fluorescent), affinity purified, specific goat anti-rabbit IgG.

Hybridomas are prepared and screened using standard techniques. Hybridomas of interest are detected by screening with labeled DRD3 to identify those fusions producing the monoclonal antibody with the desired specificity. In a typical protocol, wells of plates (FAST; Becton-Dickinson, Palo Alto, CA) are coated during incubation with affinity purified, specific rabbit anti-mouse (or suitable antispecies 1 g) antibodies at 10 mg/ml. The coated wells are blocked with 1 % bovine serum albumin, (BSA), washed and incubated with supernatants from hybridomas. After washing the wells are incubated with labeled DRD3 at 1 mg/ml. Supernatants with specific antibodies bind more labeled DRD3 than is detectable in the background. Then clones producing specific antibodies are expanded and subjected to two cycles of cloning at limiting dilution. Cloned hybridomas are injected into pristane-treated mice to produce ascites, and monoclonal antibody is purified from mouse ascitic fluid by affinity chromatography on Protein A. Monoclonal antibodies with affinities of at least

$10^8$ $M^{-1}$, preferably $10^9$ to $10^{10}$ $M^{-1}$ or stronger, are typically made by standard procedures.

**Example 8**: Diagnostic Test Using DRD3 Specific Antibodies

Particular DRD3 antibodies are useful for investigating signal transduction and the diagnosis of infectious or hereditary conditions which are characterized by differences in the amount or distribution of DRD3 or downstream products of an active signaling cascade.

Diagnostic tests for DRD3 include methods utilizing antibody and a label to detect DRD3 in human body fluids, membranes, cells, tissues or extracts of such. The polypeptides and antibodies are used with or without modification. Frequently, the polypeptides and antibodies are labeled by joining them, either covalently or noncovalently, with a substance which provides for a detectable signal. A wide variety of labels and conjugation techniques are known and have been reported extensively in both the scientific and patent literature. Suitable labels include radionuclides, enzymes, substrates, cofactors, inhibitors, fluorescent agents, chemiluminescent agents, chromogenic agents, magnetic particles and the like.

A variety of protocols for measuring soluble or membrane-bound DRD3, using either polyclonal or monoclonal antibodies specific for the protein, are known in the art. Examples include enzyme-linked immunosorbent assay (ELISA), radioimmunoassay (RIA) and fluorescent activated cell sorting (FACS). A two-site monoclonal-based immunoassay utilizing monoclonal antibodies reactive to two non-interfering epitopes on DRD3 is preferred, but a competitive binding assay may be employed.

**Example 9:** Purification of Native DRD3 Using Specific Antibodies

Native or recombinant DRD3 is purified by immunoaffinity chromatography using antibodies specific for DRD3. In general, an immunoaffinity column is constructed by covalently coupling the anti-TRH antibody to an activated chromatographic resin.

Polyclonal immunoglobulins are prepared from immune sera either by precipitation with ammonium sulfate or by purification on immobilized Protein A (Pharmacia LKB Biotechnology, Piscataway N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated Sepharose (Pharmacia LKB Biotechnology). The antibody is coupled to the resin, the resin is blocked, and the derivative resin is washed according to the manufacturer's instructions.

Such immunoaffinity columns are utilized in the purification of DRD3 by preparing a fraction from cells containing DRD3 in a soluble form. This preparation is derived by solubilization of whole cells or of a subcellular fraction obtained via differential centrifugation (with or without addition of detergent) or by other methods well known in the art. Alternatively, soluble DRD3 containing a signal sequence is secreted in useful quantity into the medium in which the cells are grown.

A soluble DRD3-containing preparation is passed over the immunoaffinity column, and the column is washed under conditions that allow the preferential absorbance of DRD3 (e.g., high ionic strength buffers in the presence of detergent). Then, the column is eluted under conditions that disrupt antibody/protein binding (e.g., a buffer of pH 2-3 or a high concentration of a chaotrope such as urea or thiocyanate ion), and DRD3 is collected.

**Example 10:** Drug Screening

This invention is particularly useful for screening therapeutic compounds by using DRD3 or binding fragments thereof in any of a variety of drug screening techniques. As DRD3 is a G protein coupled receptor any of the methods commonly used in the art may potentially be used to identify DRD3 ligands. For example, the activity of a G protein coupled receptor such as DRD3 can be measured using any of a variety of appropriate functional assays in which activation of the receptor results in an observable change in the level of some second messenger system, such as adenylate cyclase, guanylylcyclase, calcium mobilization, or inositol phospholipid hydrolysis. Alternatively, the polypeptide or fragment employed in such a test is either free in solution, affixed to a solid support, borne on a cell surface or located intracellularly. One method of drug screening utilizes eukaryotic or prokaryotic host cells which are stably transformed with recombinant nucleic acids expressing the polypeptide or fragment. Drugs are screened against such transformed cells in competitive binding assays. Such cells, either in viable or fixed form, are used for standard binding assays.

Measured, for example, is the formation of complexes between DRD3 and the agent being tested. Alternatively, one examines the diminution in complex formation between DRD3 and a ligand caused by the agent being tested.

Thus, the present invention provides methods of screening for drug canditates, drugs, or any other agents which affect signal transduction. These methods, well known in the art, comprise contacting such an agent with DRD3 polypeptide or a fragment thereof and assaying (i) for the presence of a complex between the agent and DRD3 polypeptide or fragment, or (ii) for the presence of a complex between DRD3 polypeptide or fragment and the cell. In such competitive binding assays, the DRD3 polypeptide or fragment is typically labeled. After suitable incubation, free DRD3 polypeptide or fragment is separated from that present in bound form, and the amount of free or uncomplexed label is a measure of the ability of the particular agent to bind to DRD3 or to interfere with the DRD3-agent complex.

Another technique for drug screening provides high throughput screening for compounds having suitable binding affinity to DRD3 polypeptides. Briefly stated, large numbers of different small peptide test compounds are synthesized on a solid substrate, such as plastic pins or some other surface. The peptide test compounds are reacted with DRD3 polypeptide and washed. Bound DRD3 polypeptide is then detected by methods well known in the art. Purified DRD3 are also coated directly onto plates for use in the aforementioned drug screening techniques. In addition, non-neutralizing antibodies are used to capture the peptide and immobilize it on the solid support.

This invention also contemplates the use of competitive drug screening assays in which neutralizing antibodies capable of binding DRD3 specifically compete with a test compound for binding to DRD3 polypeptides or fragments thereof. In this manner, the antibodies are used to detect the presence of any peptide which shares one or more antigenic determinants with DRD3.

**Example 11:** Rational Drug Design

The goal of rational drug design is to produce structural analogs of biologically active polypeptides of interest or of small molecules with which they interact, agonists, antagonists, or inhibitors. Any of these examples are used to fashion drugs which are more active or stable forms of the polypeptide or which enhance or interfere with the function of a polypeptide in vivo.

In one approach, the three-dimensional structure of a protein of interest, or of a protein-inhibitor complex, is determined by x-ray crystallography, by computer modeling or, most typically, by a combination of the two approaches. Both the shape and charges of the polypeptide must be ascertained to elucidate the structure and to determine active site(s) of the molecule. Less often, useful information regarding the structure of a polypeptide is gained by modeling based on the structure of homologous proteins. In both cases, relevant structural information is used to design efficient inhibitors. Useful examples of rational drug design include molecules which have improved activity or stability or which act as inhibitors, agonists, or antagonists of native peptides.

It is also possible to isolate a target-specific antibody, selected by functional assay, as described above, and then to solve its crystal structure. This approach, in principle, yields a pharmacore upon which subsequent drug design is based. It is possible to bypass protein crystallography altogether by generating anti-idiotypic antibodies (anti-ids) to a functional, pharmacologically active antibody. As a mirror image of a mirror image, the binding site of the anti-ids is expected to be an analog of the original receptor. The anti-id is then used to identify and isolate peptides from banks of chemically or biologically produced peptides. The isolated peptides then act as the pharmacore.

By virtue of the present invention, sufficient amount of polypeptide are made available to perform such analytical studies as X-ray crystallography. In addition, knowledge of the DRD3 amino acid sequence provided herein provides guidance to those employing computer modeling techniques in place of or in addition to x-ray crystallography.

**Example 12:** Identification of Other Members of the Signal Transduction Complex

The inventive purified DRD3 is a research tool for identification, characterization and purification of interacting G or other signal transduction pathway proteins. Radioactive labels are incorporated into a selected DRD3 domain by various methods known in the art and used in vitro to capture interacting molecules. A preferred method involves labeling the primary amino groups in DRD3 with $^{125}$I Bolton-Hunter reagent. This reagent has been used to label various molecules without concomitant loss of biological activity.

Labeled DRD3 is useful as a reagent for the purification of molecules with which it interacts. In one embodiment of affinity purification, membrane-bound DRD3 is covalently coupled to a chromatography column. Cell-free extract derived from synovial cells or putative target cells is passed over the column, and molecules with appropriate affinity bind to DRD3. DRD3-complex is recovered from the column, and the DRD3-binding ligand disassociated and subjected to N-terminal protein sequencing. The amino acid sequence information is then used to identify the captured molecule or to design degenerate oligonucleotide probes for cloning the relevant gene from an appropriate cDNA library.

In an alternate method, antibodies are raised against DRD3, specifically monoclonal antibodies. The monoclonal antibodies are screened to identify those which inhibit the binding of labeled DRD3. These monoclonal antibodies are then used therapeutically.

**Example 13:** Use and Administration of Antibodies, Inhibitors, or Antagonists

Antibodies, inhibitors, or antagonists of DRD3 or other treatments and compunds that are limiters of signal transduction (LSTs), provide different effects when administered therapeutically. LSTs are formulated in a nontoxic, inert, pharmaceutically acceptable aqueous carrier medium preferably at a pH of about 5 to 8, more preferably 6 to 8, although pH may vary according to the characteristics of the antibody, inhibitor, or antagonist being formulated and the condition to be treated. Characteristics of LSTs include solubility of the molecule, its half-life and antigenicity/immunogenicity. These and other characteristics aid in defining an effective carrier. Native human proteins are preferred as LSTs, but organic or synthetic molecules resulting from drug screens are equally effective in particular situations.

LSTs are delivered by known routes of administration including but not limited to topical creams and gels; transmucosal spray and aerosol; transdermal patch and bandage; injectable, intravenous and lavage formulations; and orally administered liquids and pills particularly formulated to resist stomach acid and enzymes. The particular formulation, exact dosage, and route of administration is determined by the attending physician and varies according to each specific situation.

Such determinations are made by considering multiple variables such as the condition to be treated, the LST to be administered, and the pharmacokinetic profile of a particular LST. Additional factors which are taken into account include severity of the disease state, patient's age, weight, gender and diet, time and frequency of LST administration, possible combination with other drugs, reaction sensitivities, and tolerance/response to therapy. Long acting LST formulations might be administered every 3 to 4 days, every week, or once every two weeks depending on half-life and clearance rate of the particular LST.

Normal dosage amounts vary from 0.1 to $10^5$ μg, up to a total dose of about 1 g, depending upon the route of administration. Guidance as to particular dosages and methods of delivery is provided in the literature; see U.S. Pat. Nos. 4,657,760; 5,206,344; or 5,225,212. Those skilled in the art employ different formulations for different LSTs. Administration to cells such as nerve cells necessitates delivery in a manner different from that to other cells such as vascular endothelial cells.

It is contemplated that abnormal signal transduction, trauma, or diseases which trigger DRD3 activity are treatable with LSTs. These conditions or diseases are specifically diagnosed by the tests discussed above, and such testing should be performed in suspected cases of viral, bacterial or fungal infections, allergic responses, mechanical injury associated with trauma, hereditary diseases, lymphoma or carcinoma, or other conditions which activate the genes of lymphoid or neuronal tissues.

**Example 14**: Production of Non-human Transgenic Animals

Animal model systems which elucidate the physiological and behavioral roles of the DRD3 are produced by creating nonhuman transgenic animals in which the activity of the DRD3 is either increased or decreased, or the amino acid sequence of the expressed DRD3 is altered, by a variety of techniques. Examples of these techniques include, but are not limited to: 1) Insertion of normal or mutant versions of DNA encoding a DRD3, by microinjection, electroporation, retroviral transfection or other means well known to those skilled in the art, into appropriately fertilized embryos in order to produce a transgenic animal or 2) homologous recombination of mutant or normal, human or animal versions of these genes with the native gene locus in transgenic animals to alter the regulation of expression or the structure of these DRD3 sequences. The technique of homologous recombination is well known in the art. It replaces the native gene with the inserted gene and hence is useful for producing an animal that cannot express native DRD3s but does express, for example, an inserted mutant DRD3, which has replaced the native DRD3 in the animal's genome by recombination, resulting in underexpression of the transporter. Microinjection adds genes to the genome, but does not remove them, and the technique is useful for producing an animal which expresses its own and added DRD3, resulting in overexpression of the DRD3.

One means available for producing a transgenic animal, with a mouse as an example, is as follows: Female mice are mated, and the resulting fertilized eggs are dissected out of their oviducts. The eggs are stored in an appropriate medium such as cesium-chloride M2 medium. DNA or cDNA encoding DRD3 is purified from a vector by methods well known to the one skilled in the art. Inducible promoters may be fused with the coding region of the DNA to provide an experimental means to regulate expression of the transgene. Alternatively or in addition, tissue specific regulatory elements may be fused with the coding region to permit tissue-specific expression of the transgene. The DNA, in an appropriately buffered solution, is put into a microinjection needle (which may be made from capillary tubing using a piper puller) and the egg to be injected is put in a depression slide. The needle is inserted into the pronucleus of the egg, and the DNA solution is injected. The injected egg is then transferred into the oviduct of a pseudopregnant mouse which is a mouse stimulated by the appropriate hormones in order to maintain false pregnancy, where it proceeds to the uterus, implants, and develops to term. As noted above, microinjection is not the only method for inserting DNA into the egg but is used here only for exemplary purposes.

**References**

WO 03094856

U.S. 4,522,811
U.S. 5,283,317
U.S. 5,407,823
U.S. 5,422,265
U.S. 5,508,384
U.S. 5,565,332
U.S. 5,594,108
WO 84/03564
WO 92/01810
WO 92/07937
WO 93/03151
WO 94/13804
WO 01/04297
WO 01/77172
Accili et al., Proc. Nat. Acad. Sci. 93:1945-1949, 1996
Altschul SF, Madden TL, Schaffer AA, Zhang J, Zhang Z, Miller W, Lipman DJ; Nucleic Acids Res 1997 Sep 1; 25 (17): 3389-402
Appa Rao et al., 1997, Protein Expr PurifNov, 11 (2): 201-8
Asico et al., J. Clin. Invest. 102: 493-498, 1998
Barnes, 2000, Chest, 117:10S14S
Botstein et al., 1980, Am J Hum Genet. 32: 314-31
Colbere-Garapin et al., 1981, J. Mol. Biol. 150, 1-14
Crocq et al., J. Med. Genet. 29: 858-860,1992
Engelhard et al., 1994, Proc. Nat. Acad. Sci. 91, 3224-3227
Gergen and Weiss , 1992, Am Rev Respir Dis 146:823-824
Gibson et al., 1996, Genome Research 6: 995-1001
Guillin et al., Nature 411: 86-89, 2001
Haseloff et al., 1988 , Nature 334, 585-591
Heid et al., 1996, Genome Research 6: 986-994
Holland et al., 1991, PNAS 88: 7276-7280
Holzman, Brain Res. Rev. 31: 350-356, 2000
Ilani et al., Proc. Nat. Acad. Sci. 98: 625-628, 2001
Iwabuchi et al., 1993, Oncogene 8, 1693-1696
Jeffreys et al., 1985, Nature 316: 76-9
Johnson et al., 1989, Endoc. Rev. 10, 317-331
Kellogg et al., 1990, Anal. Biochem. 189:202-208
Lam, 1997, Anticancer Drug Res. 12(3):145-67
Le Coniat et al., Hum. Genet. 87: 618-620, 1991
Lefkowitz, 1991 , Nature 351, 353-.354
Livak et al., 1995, PCR Methods and Applications 357-362
Logan, Shenk, 1984, Proc. Natl. Acad Sci. 81, 3655-3659
Lohmueller et al., Nature Genet. 33: 177-182, 2003
Lowy et al., 1980, Cell 22, 817-23
Maddox et al., 1983, J. Exp. Med 158, 1211-1216
Mc Allister et al., J. Recept Signal Transduction Res. 15:267-81, 1995
McConnell et al., 1992, Science 257, 1906-1912
Nanko et al., Hum. Genet 92: 336-338, 1993
Nicholls et al., 1993, J. Immunol. Meth. 165, 81-91
Nothen et al., J. Med. Genet. 30: 708-712,1993
Piatak et al., 1993, BioTechniques 14:70-81
Piatak et al., 1993, Science 259:1749-1754
Pilla et al., Nature 400: 371-375, 1999
Porath et al., 1992, Prot. Exp. Purif. 3, 263-281
Roberge et al., 1995, Science 269, 202-204
Rybakowski et al., Molec. Psychiat. 6: 718-724, 2001
Shaikh et al., J. Med. Genet. 30: 308-309,1993
Sjolander, Urbaniczky, 1991, Anal. Chem. 63, 2338-2345
Sokoloff et al., Nature 347:146-151,1990

Spurlock et al., Am. J. Med. Genet. 81: 24-28, 1998
Szabo et al., 1995, Curr. Opin. Struct. Biol. 5, 699-705
Thomas, 1980, Proc. Nat. Acad. Sci., 77:5201-5205
Uhlmann et al., 1987, Tetrahedron. Lett. 215, 3539-3542
Weber et al., 1990, Genomics 7: 524-30
Wigler et al., 1977, Cell 11, 223-32
Wigler et al., 1980, Proc. Natl. Acad Sci., 77, 3567-70

## Claims

1. An in vitro method of screening for therapeutic agents useful in the treatment of a disease comprised in a group of diseases consisting of heart failure, ischemic diseases of the heart, myocardial infarction and peripheral vascular diseases in a mammal comprising the steps of

   i) determining the activity of a DRD3 polypeptide at a certain concentration of a test compound or in the absence of said test compound,
   ii) determining the activity of said polypeptide at a different concentration of said test compound.

2. An in vitro method of screening for therapeutic agents useful in the treatment of a disease comprised in a group of diseases consisting of heart failure, ischemic diseases of the heart, myocardial infarction and peripheral vascular diseases in a mammal comprising the steps of

   i) determining the activity of a DRD3 polypeptide at a certain concentration of a test compound,
   ii) determining the activity of a DRD3 polypeptide at the presence of a compound known to be a regulator of a DRD3 polypeptide.

3. An in vitro method of screening for therapeutic agents useful in the treatment of a disease comprised in a group of diseases consisting of heart failure, ischemic diseases of the heart, myocardial infarction and peripheral vascular diseases in a mammal preceding the methods of claim 1 or 2 and comprising the steps of

   i) contacting a test compound with a DRD3 polypeptide,
   ii) detect binding of said test compound to said DRD3 polypeptide.

4. The method of any of claims 1 to 3, wherein the step of contacting is in or at the surface of a cell.

5. The method of any of claims 1 to 3, wherein the cell is in vitro.

6. The method of any of claims 1 to 3, wherein the step of contacting is in a cell-free system.

7. The method of any of claims 1 to 3, wherein the polypeptide is coupled to a detectable label.

**8.** The method of any of claims 1 to 3, wherein the compound is coupled to a detectable label.

**9.** The method of any of claims 1 to 3, wherein the test compound displaces a ligand which is first bound to the polypeptide.

**10.** The method of any of claims 1 to 3, wherein the polypeptide is attached to a solid support.

**11.** The method of any of claims 1 to 3, wherein the compound is attached to a solid support.

**12.** An in vitro method of screening for therapeutic agents useful in the treatment of a disease comprised in a group of diseases consisting of heart failure, ischemic diseases of the heart, myocardial infarction and peripheral vascular diseases in a mammal comprising the steps of

i) contacting a test compound with a DRD3 polynucleotide,
ii) detect binding of said test compound to said DRD3 polynucleotide.

**13.** The method of claim 12 wherein the nucleic acid molecule is RNA.

**14.** The method of claim 12 wherein the contacting step is in or at the surface of a cell.

**15.** The method of claim 12 wherein the contacting step is in a cell-free system.

**16.** The method of claim 12 wherein polynucleotide is coupled to a detectable label.

**17.** The method of claim 12 wherein the test compound is coupled to a detectable label.

**18.** Use of

i) an antisense oligonucleotide,
ii) an antibody, or
iii) a ribozyme.

which regulate the activity of a DRD3 polypeptide according to any claim 1 - 17 for the preparation of a medicament useful in the treatment of a disease comprised in a group of diseases consisting of heart failure, ischemic diseases of the heart, myocardial infarction and peripheral vascular diseases in a mammal.

**19.** Use of a DRD3 polynucleotide for the preparation of a medicament useful in the treatment of a disease comprised in a group of diseases consisting of ischemic diseases of the heart, myocardial infarction, atherosclerosis and peripheral vascular diseases in a mammal.

**20.** Use of a DRD3 polypeptide for the preparation of a medicament useful in the treatment of a disease comprised in a group of diseases consisting of heart failure, ischemic diseases of the heart, myocardial infarction and peripheral vascular diseases in a mammal.

**Patentansprüche**

**1.** In-vitro-Verfahren zum Screening nach therapeutischen Mitteln, die in der Behandlung einer Erkrankung aus einer Gruppe von Erkrankungen, welche Herzinsuffizienz, ischämische Herzerkrankungen, Myokardinfarkt und periphere Gefäßerkrankungen umfasst, bei einem Säugetier von Nutzen sind, umfassend folgende Schritte:

i) die Bestimmung der Aktivität eines DRD3-Polypeptids bei einer gewissen Konzentration einer Testverbindung oder in Abwesenheit der Testverbindung,
ii) die Bestimmung der Aktivität des Polypeptids bei einer anderen Konzentration der Testverbindung.

**2.** In-vitro-Verfahren zum Screening nach therapeutischen Mitteln, die in der Behandlung einer Erkrankung aus einer Gruppe von Erkrankungen, welche Herzinsuffizienz, ischämische Herzerkrankungen, Myokardinfarkt und periphere Gefäßerkrankungen umfasst, bei einem Säugetier von Nutzen sind, umfassend folgende Schritte:

i) die Bestimmung der Aktivität eines DRD3-Polypeptids bei einer gewissen Konzentration einer Testverbindung,
ii) die Bestimmung der Aktivität eines DRD3-Polypeptids in Gegenwart einer Verbindung, von der bekannt ist, dass sie ein Regulator eines DRD3-Polypeptids ist.

**3.** In-vitro-Verfahren zum Screening nach therapeutischen Mitteln, die in der Behandlung einer Erkrankung aus einer Gruppe von Erkrankungen, welche Herzinsuffizienz, ischämische Herzerkrankungen, Myokardinfarkt und periphere Gefäßerkrankungen umfasst, bei einem Säugetier von Nutzen sind, das dem Verfahren nach Anspruch 1 oder 2 vorausgeht, umfassend folgende Schritte:

i) das Kontaktieren einer Testverbindung mit einem DRD3-Polypeptid,
ii) das Detektieren der Bindung der Testverbindung an das DRD3-Polypeptid.

**4.** Verfahren nach einem der Ansprüche 1 bis 3, worin der Schritt des Kontaktierens in einer oder an der Oberfläche einer Zelle stattfindet.

**5.** Verfahren nach einem der Ansprüche 1 bis 3, worin die Zelle in vitro vorliegt.

**6.** Verfahren nach einem der Ansprüche 1 bis 3, worin der Schritt des Kontaktierens in einem zellfreien System erfolgt.

**7.** Verfahren nach einem der Ansprüche 1 bis 3, worin das Polypeptid an eine detektierbare Markierung gebunden ist.

**8.** Verfahren nach einem der Ansprüche 1 bis 3, worin die Verbindung an eine detektierbare Markierung gekoppelt ist.

**9.** Verfahren nach einem der Ansprüche 1 bis 3, worin die Testverbindung einen Liganden verdrängt, der zuerst an das Polypeptid gebunden ist.

**10.** Verfahren nach einem der Ansprüche 1 bis 3, worin das Polypeptid an einen festen Träger gebunden ist.

**11.** Verfahren nach einem der Ansprüche 1 bis 3, worin die Verbindung an einen festen Träger gebunden ist.

**12.** In-vitro-Verfahren zum Screening nach therapeutischen Mitteln, die in der Behandlung einer Erkrankung aus einer Gruppe von Erkrankungen, welche Herzinsuffizienz, ischämische Herzerkrankungen, Myokardinfarkt und periphere Gefäßerkrankungen umfasst, bei einem Säugetier von Nutzen sind, umfassend folgende Schritte:

i) das Kontaktieren einer Testverbindung mit einem DRD3-Polynucleotid,
ii) das Detektieren der Bindung der Testverbindung an das DRD3-Polynucleotid.

**13.** Verfahren nach Anspruch 12, worin das Nucleinsäuremolekül RNA ist.

**14.** Verfahren nach Anspruch 12, worin der Schritt des Kontaktierens in einer oder an der Oberfläche einer Zelle erfolgt.

**15.** Verfahren nach Anspruch 12, worin der Schritt des Kontaktierens in einem zellfreien System erfolgt.

**16.** Verfahren nach Anspruch 12, worin das Polynucleotid an eine detektierbare Markierung gebunden ist.

**17.** Verfahren nach Anspruch 12, worin die Testverbindung an eine detektierbare Markierung gebunden ist.

**18.** Verwendung

i) eines Antisense-Oligonucleotids,
ii) eines Antikörpers oder
iii) eines Ribozyms,

welche die Aktivität eines DRD3-Polypeptids nach einem der Ansprüche 1 bis 17 steuern, zur Herstellung eines Medikaments, das in der Behandlung einer Erkrankung aus einer Gruppe von Erkrankungen, welche Herzinsuffizienz, ischämische Herzerkrankungen, Myokardinfarkt und periphere Gefäßerkrankungen umfasst, bei einem Säugetier von Nutzen ist.

**19.** Verwendung eines DRD3-Polynucleotids zur Herstellung eines Medikaments, das in der Behandlung einer Erkrankung aus einer Gruppe von Erkrankungen, welche ischämische Herzerkrankungen, Myokardinfarkt, Atherosklerose und periphere Gefäßerkrankungen umfasst, bei einem Säugetier von Nutzen ist.

**20.** Verwendung eines DRD3-Polypeptids zur Herstellung eines Medikaments, das in der Behandlung einer Erkrankung aus einer Gruppe von Erkrankungen, welche Herzinsuffizienz, ischämische Herzerkrankungen, Myokardinfarkt und periphere Gefäßerkrankungen umfasst, bei einem Säugetier von Nutzen ist.

## Revendications

**1.** Procédé de criblage in vitro d'agents thérapeutiques utiles dans le traitement d'une maladie comprise parmi un groupe de maladies constitué de l'insuffisance cardiaque, de cardiopathies ischémiques, de l'infarctus du myocarde et de maladies vasculaires périphériques chez un mammifère comprenant les étapes de :

i) détermination de l'activité d'un polypeptide DRD3 à une certaine concentration d'un composé test ou en l'absence dudit composé test,
ii) détermination de l'activité dudit polypeptide à une concentration différente dudit composé test.

**2.** Procédé de criblage in vitro d'agents thérapeutiques utiles dans le traitement d'une maladie comprise parmi un groupe de maladies constitué de l'insuffisance cardiaque, de cardiopathies ischémiques, de l'infarctus du myocarde et de maladies vasculaires périphériques chez un mammifère comprenant les étapes de :

i) détermination de l'activité d'un polypeptide DRD3 à une certaine concentration d'un composé test,
ii) détermination de l'activité d'un polypeptide DRD3 en présence d'un composé reconnu comme étant un régulateur d'un polypeptide DRD3.

**3.** Procédé de criblage in vitro d'agents thérapeutiques utiles dans le traitement d'une maladie comprise parmi un groupe de maladies constitué de l'insuffisance cardiaque, des cardiopathies ischémiques, de l'infarctus du myocarde et des maladies vasculaires périphériques chez un mammifère précédant les procédés selon la revendication 1 ou 2 et comprenant les étapes de :

i) mise en contact d'un composé test avec un polypeptide DRD3,
ii) détection de la liaison dudit composé test audit polypeptide DRD3.

**4.** Procédé selon l'une quelconque des revendications 1 à 3, dans lequel l'étape de mise en contact se fait dans une cellule ou à la surface d'une cellule.

**5.** Procédé selon l'une quelconque des revendications 1 à 3, dans lequel la cellule est in vitro.

**6.** Procédé selon l'une quelconque des revendications 1 à 3, dans lequel l'étape de mise en contact se fait dans un système sans cellule.

**7.** Procédé selon l'une quelconque des revendications 1 à 3, dans lequel le polypeptide est couplé à un traceur détectable.

**8.** Procédé selon l'une quelconque des revendications 1 à 3, dans lequel le composé est couplé à un traceur détectable.

**9.** Procédé selon l'une quelconque des revendications 1 à 3, dans lequel le composé test déplace un ligand qui est d'abord lié au polypeptide.

**10.** Procédé selon l'une quelconque des revendications 1 à 3, dans lequel le polypeptide est attaché à un support solide.

**11.** Procédé selon l'une quelconque des revendications 1 à 3, dans lequel le composé est attaché à un support solide.

**12.** Procédé de criblage in vitro d'agents thérapeutiques utiles dans le traitement d'une maladie comprise parmi un groupe de maladies constitué de l'insuffisance cardiaque, de cardiopathies ischémiques, de l'infarctus du myocarde et de maladies vasculaires périphériques chez un mammifère comprenant les étapes de :

i) mise en contact d'un composé test avec un polynucléotide DRD3,
ii) détection de la liaison dudit composé test audit polynucléotide DRD3.

**13.** Procédé selon la revendication 12 dans lequel la molécule d'acide nucléique est un ARN.

**14.** Procédé selon la revendication 12 dans lequel l'étape de mise en contact se fait dans une cellule ou à la surface d'une cellule.

**15.** Procédé selon la revendication 12 dans lequel l'étape de mise en contact se fait dans un système sans cellule.

**16.** Procédé selon la revendication 12 dans lequel le polynucléotide est couplé à un traceur détectable.

**17.** Procédé selon la revendication 12 dans lequel le composé test est couplé à un traceur détectable.

**18.** Utilisation

i) d'un oligonucléotide antisens,
ii) d'un anticorps, ou
iii) d'un ribozyme,

qui régule l'activité d'un polypeptide DRD3 selon l'une quelconque des revendications 1-17 pour la préparation d'un médicament utile dans le traitement d'une maladie comprise parmi un groupe de maladies constitué de l'insuffisance cardiaque, de cardiopathies ischémiques, de l'infarctus du myocarde et de maladies vasculaires périphériques chez un mammifère.

**19.** Utilisation d'un polynucléotide DRD3 pour la préparation d'un médicament utile dans le traitement d'une maladie comprise parmi un groupe de maladies constitué de l'insuffisance cardiaque, de cardiopathies ischémiques, de l'infarctus du myocarde et de maladies vasculaires périphériques chez un mammifère.

**20.** Utilisation d'un polypeptide DRD3 pour la préparation d'un médicament utile dans le traitement d'une maladie comprise parmi un groupe de maladies constitué de l'insuffisance cardiaque, de cardiopathies ischémiques, de l'infarctus du myocarde et de maladies vasculaires périphériques chez un mammifère.

**Fig. 1**

SEQ ID NO: 1

ATGGCATCTCTGAGTCAGCTGAGTAGCCACCTGAACTACACCTGTGGG
GCAGAGAACTCCACAGGTGCCAGCCAGGCCCGCCCACATGCCTACTAT
GCCCTCTCCTACTGCGCGCTCATCCTGGCCATCGTCTTCGGCAATGGC
CTGGTGTGCATGGCTGTGCTGAAGGAGCGGGCCCTGCAGACTACCACC
AACTACTTAGTAGTGAGCCTGGCTGTGGCAGACTTGCTGGTGGCCACC
TTGGTGATGCCCTGGGTGGTATACCTGGAGGTGACAGGTGGAGTCTGG
AATTTCAGCCGCATTTGCTGTGATGTTTTTGTCACCCTGGATGTCATG
ATGTGTACAGCCAGCATCCTTAATCTCTGTGCCATCAGCATAGACAGG
TACACTGCAGTGGTCATGCCCGTTCACTACCAGCATGGCACGGGACAG
AGCTCCTGTCGGCGCGTGGCCCTCATGATCACGGCCGTCTGGGTACTG
GCCTTTGCTGTGTCCTGCCCTCTTCTGTTTGGCTTTAATACCACAGGG
GACCCCACTGTCTGCTCCATCTCCAACCCTGATTTTGTCATCTACTCT
TCAGTGGTGTCCTTCTACCTGCCCTTTGGAGTGACTGTCCTTGTCTAT
GCCAGAATCTATGTGGTGCTGAAACAAAGGAGACGGAAAAGGATCCTC
ACTCGACAGAACAGTCAGTGCAACAGTGTCAGGCCTGGCTTCCCCCAA
CAAACCCTCTCTCCTGACCCGGCACATCTGGAGCTGAAGCGTTACTAC
AGCATCTGCCAGGACACTGCCTTGGGTGGACCAGGCTTCCAAGAAAGA
GGAGGAGAGTTGAAAAGAGAGGAGAAGACTCGGAATTCCCTGAGTCCC
ACCATAGCGCCCAAGCTCAGCTTAGAAGTTCGAAAACTCAGCAATGGC
AGATTATCGACATCTTTGAAGCTGGGGCCCCTGCAACCTCGGGGAGTG
CCACTTCGGGAGAAGAAGGCAACCCAAATGGTGGCCATTGTGCTTGGG
GCCTTCATTGTCTGCTGGCTGCCCTTCTTCTTGACCCATGTTCTCAAT
ACCCACTGCCAGACATGCCACGTGTCCCCAGAGCTTTACAGTGCCACG
ACATGGCTGGGCTACGTGAATAGCGCCCTCAACCCTGTGATCTATACC
ACCTTCAATATCGAGTTCCGGAAAGCCTTCCTCAAGATCCTGTCTTGC
TGA

**Fig. 2**

SEQ ID NO: 2

MASLSQLSSHLNYTCGAENSTGASQARPHAYYALSYCALILAIVFGNG
LVCMAVLKERALQTTTNYLVVSLAVADLLVATLVMPWVVYLEVTGGVW
NFSRICCDVFVTLDVMMCTASILNLCAISIDRYTAVVMPVHYQHGTGQ
SSCRRVALMITAVWVLAFAVSCPLLFGFNTTGDPTVCSISNPDFVIYS
SVVSFYLPFGVTVLVYARIYVVLKQRRRKRILTRQNSQCNSVRPGFPQ
QTLSPDPAHLELKRYYSICQDTALGGPGFQERGGELKREEKTRNSLSP
TIAPKLSLEVRKLSNGRLSTSLKLGPLQPRGVPLREKKATQMVAIVLG
AFIVCWLPFFLTHVLNTHCQTCHVSPELYSATTWLGYVNSALNPVIYT
TFNIEFRKAFLKILSC

**Fig. 3**

SEQ ID NO: 3

5'- AAACCCTCTCTCCTGACCCG -3'

**Fig. 4**

SEQ ID NO: 4

5'- ACCCAAGGCAGTGTCCTGG -3'

**Fig. 5**

SEQ ID NO: 5

5'- CACATCTGGAGCTGAAGCGTTACTACAGCA -3'

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 920793 A
- WO 200177172 A
- WO 03094856 A
- WO 9207937 A
- US 5565332 A
- WO 9303151 A
- WO 9413804 A
- US 5641673 A
- US 5283317 A
- WO 9201810 A
- US 4522811 A
- WO 8403564 A
- US 5407823 A
- US 5594108 A
- US 5422265 A
- US 5508384 A
- US 4657760 A
- US 5206344 A
- US 5225212 A
- WO 0104297 A
- WO 0177172 A


### Non-patent literature cited in the description


- **PIATAK.** *BioTechniques,* 1993
- **PIATAK.** *Science,* 1993
- **SCOTT ; SMITH.** *Science,* 1990, vol. 249, 386-390
- **ALTSCHUL SF ; MADDEN TL ; SCHAFFER AA ; ZHANG J ; ZHANG Z ; MILLER W ; LIPMAN DJ.** *Nucleic Acids Res,* 01 September 1997, vol. 25 (17), 3389-402
- **KOBILKA et al.** *Science,* 1988, vol. 240, 1310-1316
- **ACCILI et al.** *Proc. Nat. Acad. Sci.,* 1996, vol. 93, 1945-1949
- **APPA RAO et al.** *Protein Expr PurifNov,* 1997, vol. 11 (2), 201-8
- **ASICO et al.** *J. Clin. Invest.,* 1998, vol. 102, 493-498
- **BARNES.** *Chest,* 2000, vol. 117, 10S14S
- **BOTSTEIN et al.** *Am J Hum Genet.,* 1980, vol. 32, 314-31
- **COLBERE-GARAPIN et al.** *J. Mol. Biol.,* 1981, vol. 150, 1-14
- **CROCQ et al.** *J. Med. Genet.,* 1992, vol. 29, 858-860
- **ENGELHARD et al.** *Proc. Nat. Acad. Sci.,* 1994, vol. 91, 3224-3227
- **GERGEN ; WEISS.** *Am Rev Respir Dis,* 1992, vol. 146, 823-824
- **GIBSON et al.** *Genome Research,* 1996, vol. 6, 995-1001
- **GUILLIN et al.** *Nature,* 2001, vol. 411, 86-89
- **HASELOFF et al.** *Nature,* 1988, vol. 334, 585-591
- **HEID et al.** *Genome Research,* 1996, vol. 6, 986-994
- **HOLLAND et al.** *PNAS,* 1991, vol. 88, 7276-7280
- **HOLZMAN.** *Brain Res. Rev.,* 2000, vol. 31, 350-356
- **ILANI et al.** *Proc. Nat. Acad. Sci.,* 2001, vol. 98, 625-628
- **IWABUCHI et al.** *Oncogene,* 1993, vol. 8, 1693-1696
- **JEFFREYS et al.** *Nature,* 1985, vol. 316, 76-9
- **JOHNSON et al.** *Endoc. Rev.,* 1989, vol. 10, 317-331
- **KELLOGG et al.** *Anal. Biochem.,* 1990, vol. 189, 202-208
- **LAM.** *Anticancer Drug Res.,* 1997, vol. 12 (3), 145-67
- **LE CONIAT et al.** *Hum. Genet.,* 1991, vol. 87, 618-620
- **LEFKOWITZ.** *Nature,* 1991, vol. 351, 353-.354
- **LIVAK et al.** *PCR Methods and Applications,* 1995, 357-362
- **LOGAN, SHENK.** *Proc. Natl. Acad Sci.,* 1984, vol. 81, 3655-3659
- **LOHMUELLER et al.** *Nature Genet.,* 2003, vol. 33, 177-182
- **LOWY et al.** *Cell,* 1980, vol. 22, 817-23
- **MADDOX et al.** *J. Exp. Med,* 1983, vol. 158, 1211-1216
- **MC ALLISTER et al.** *J. Recept Signal Transduction Res.,* 1995, vol. 15, 267-81
- **MCCONNELL et al.** *Science,* 1992, vol. 257, 1906-1912
- **NANKO et al.** *Hum. Genet,* 1993, vol. 92, 336-338

- **NICHOLLS et al.** *J. Immunol. Meth.,* 1993, vol. 165, 81-91
- **NOTHEN et al.** *J. Med. Genet.,* 1993, vol. 30, 708-712
- **PIATAK et al.** *BioTechniques,* 1993, vol. 14, 70-81
- **PIATAK et al.** *Science,* 1993, vol. 259, 1749-1754
- **PILLA et al.** *Nature,* 1999, vol. 400, 371-375
- **PORATH et al.** *Prot. Exp. Purif.,* 1992, vol. 3, 263-281
- **ROBERGE et al.** *Science,* 1995, vol. 269, 202-204
- **RYBAKOWSKI et al.** *Molec. Psychiat.,* 2001, vol. 6, 718-724
- **SHAIKH et al.** *J. Med. Genet.,* 1993, vol. 30, 308-309
- **SJOLANDER, URBANICZKY.** *Anal. Chem.,* 1991, vol. 63, 2338-2345
- **SOKOLOFF et al.** *Nature,* 1990, vol. 347, 146-151
- **SPURLOCK et al.** *Am. J. Med. Genet.,* 1998, vol. 81, 24-28
- **SZABO et al.** *Curr. Opin. Struct. Biol.,* 1995, vol. 5, 699-705
- **THOMAS.** *Proc. Nat. Acad. Sci.,* 1980, vol. 77, 5201-5205
- **UHLMANN et al.** *Tetrahedron. Lett.,* 1987, vol. 215, 3539-3542
- **WEBER et al.** *Genomics,* 1990, vol. 7, 524-30
- **WIGLER et al.** *Cell,* 1977, vol. 11, 223-32
- **WIGLER et al.** *Proc. Natl. Acad Sci.,* 1980, vol. 77, 3567-70